# EUROPÄISCHE PATENTSCHRIFT

(11) **EP 3 230 404 B1**
(45) Veröffentlichungstag und Bekanntmachung des Hinweises auf die Patenterteilung: **05.02.2020**
(21) Anmeldenummer: 15825601.6
(22) Anmeldetag: 08.12.2015
(51) Int. Cl.: C09K 11/06, H01L 51/50

(54) **AMBIPOLARE HOSTMATERIALIEN UND DEREN VERWENDUNG**
AMBIPOLAR HOST MATERIALS AND THEIR USE
MATÉRIAUX HÔTES AMBIPOLAIRES ET LEUR UTILISATION

(30) Priorität: 09.12.2014 EP 14196901
(43) Veröffentlichungstag der Anmeldung: 18.10.2017
(73) Patentinhaber: cynora GmbH, 76646 Bruchsal (DE)
(72) Erfinder: GEORGIOS, Liaptsis, 68161 Mannheim (DE)
(74) Vertreter: Hoppe, Georg Johannes
(86) Internationale Anmeldenummer: PCT/EP2015/078997
(87) Internationale Veröffentlichungsnummer: WO 2016/091887

(56) Entgegenhaltungen:
- WO-A1-2011/132865
- WO-A1-2012/056966
- WO-A1-2014/071836
- CN-A- 101 747 289
- CN-A- 102 372 670
- CN-A- 103 772 291
- JP-A- 2000 095 766
- JP-A- 2007 262 134
- US-A1- 2012 165 523
- US-A1- 2014 225 070
- US-A1- 2014 343 293

## Beschreibung

Die Erfindung betrifft Moleküle der allgemeinen Formel 1, und deren Verwendung als Hostmaterialien in OLEDs (organic light emitting diodes) und anderen opto-elektronischen Bauelementen.

### Einleitung

Die auf OLED basierende Technologie hat sich in den letzten Jahren im Bereich von Beleuchtungs- und Displayapplikationen als kostengünstige und energieeffiziente Technik durchgesetzt. Entsprechende Produkte sind bereits heute kommerziell erhältlich und werden in verschiedensten Arten von opto-elektronischen Bauelementen eingesetzt.

Der Aufbau organischer Elektrolumineszenzvorrichtungen wie z. B. OLEDs, in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Steigerung der Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer. Dies gilt insbesondere für OLEDs, welche im kürzerwelligen Bereich des für den Menschen sichtbaren Spektrums, beispielsweise grün oder blau, emittieren.

Die Eigenschaften von phosphoreszierenden OLEDs werden nicht nur von den eingesetzten Triplettemittern bestimmt. Hier sind insbesondere auch die anderen verwendeten Materialien, wie Matrixmaterialien (auch Host oder Wirt genannt), Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf. Gemäß dem Stand der Technik werden unter anderem Ketone (z. B. gemäß WO 04/093207 A oder WO 10/006680 A) oder Phosphinoxide (z. B. gemäß WO 05/003253 A) als Matrixmaterialien für phosphoreszierende Emitter verwendet. Allerdings besteht bei Verwendung dieser Matrixmaterialien ebenso wie bei anderen Matrixmaterialien noch Verbesserungsbedarf, insbesondere in Bezug auf die Effizienz und die Lebensdauer der Vorrichtung.

Die Emissionsschicht besteht in hocheffizienten OLEDs aus einem Wirt-Gast System, worin der Gast den Emitter darstellt, der Wirt als Ladungs- und Energietransfermaterial dient. Herzstück solcher Bauteile ist die Emitterschicht, welche typischerweise mit einer Anode und einer Kathode in Kontakt gebracht werden und die in der Regel aus einem Hostmaterial besteht, in welches die emittierenden Moleküle eingebettet sind. Weitere Schichten, wie die Lochinjektionsschicht, die Lochtransportschicht, die Elektronenleitschicht und die Zwischenschicht bewirken im Allgemeinen eine dramatisch verbesserte Effizienz der Bauteile. Bei Anlegen eines Stroms/einer Spannung treffen sich negative Ladungsträger (Elektronen) und positive Ladungsträger (Löcher), die zu sogenannten Exzitonen (= angeregte Zustände) rekombinieren. Die in den Exzitonen enthaltene Energie kann von den entsprechenden Emittern in Form von Licht abgegeben werden, wobei man in diesem Fall von Elektrolumineszenz spricht. Einen Überblick über die Funktion von OLEDs findet sich beispielsweise bei H. Yersin, Top. Curr. Chem. 2004, 241, 1 und H. Yersin, "Highly Efficient OLEDs with Phosphorescent Materials"; Wiley-VCH, Weinheim, Germany, 2008.

Alle Materialien eines opto-elektronischen Bauelements müssen so gewählt werden, dass eine optimale Injektion der Ladungsträger (Löcher und Elektronen) von den Elektroden in das optoelektronische Material, sowie deren feldunterstützter Transport hin zur Emitterschicht unter dortiger Ausbildung der Exzitonen ermöglicht wird. Die Entwicklung effektiver Hostmaterialien ist für die Produktion effizienter opto-elektronischer Bauelemente hierbei genau so wichtig wie die Entwicklung neuer Emittermaterialien.

Hostmaterialien müssen eine effiziente Energieniveau-Nivellierung mit den angrenzenden Schichten ermöglichen. Die Energieniveaus der verwendeten Elektroden werden hierbei unter Verwendung von Injektions-, Transport- und Blockermaterialien stufenweise an die Energieniveaus der Emissionsschicht, folglich dem Wirt und dem Emittermolekül, angepasst. Weiterhin sollten die verwendeten Hostmaterialien idealerweise einen ausgeglichenen Ladungstransport (eine Ladungsträgerbalance von positiven und negativen Ladungsträgern) gewährleisten. Gängige Hostmaterialien, wie CBP (4,4'-bis(N-carbazolyl)biphenyl), mCP (1,3-bis(9-carbazolyl)benzene), TPBi (1,3,5-tris(N-phenylbenzimidazol-2-yl)benzene) und TCTA (4-(9H-Carbazol-9-yl)-N,N-bis[4-(9H-carbazol-9-yl)phenyl]aniline) sind jedoch nur in der Lage, entweder bevorzugt Löcher oder bevorzugt Elektronen zu transportieren. Beim Einsatz solcher unipolaren Materialien findet die Ladungsträgerrekombination und Bildung der Exzitonen meist nahe der Grenzfläche zur Ladungstransport-Schicht statt. Diese räumliche Beschränkung kann bei wachsender Helligkeit oder Stromdichte zu einer Effizienz-Abnahme der optoelektronischen Bauteile ("roll-off") führen. Durch die schmale Rekombinationszone und die dadurch bedingte hohe Excitonendichte in einem kleinen Bereich der Emissionsschicht werden Degradationsprozesse in dieser Region der Emissionsschicht beschleunigt, wodurch das Bauteil keine optimale Betriebsdauer erreichen kann. Dieses Problem kann durch den Einsatz von ambipolaren Hosts für OLEDs in der Emissionsschicht umgangen werden. Dabei werden zum einen Donor- und Akzeptormoleküle als Mischungen eingesetzt (K. Goushi, K. Yoshida, K. Sato, C. Adachi, Nat. Photonics 2012, 6, 253-258), welche die Effizienz der Bauteile vor allem bei hohen Stromdichten nachhaltig verbessern, da die Emissionszone innerhalb der Emissionsschicht verbreitert wird und die Energiedichte dadurch herabgesetzt wird. Allerdings führen die benötigten hohen Konzentrationen des zusätzlichen Ladungstransportmaterials häufig zu Phasentrennungen und dadurch zu Verschlechterungen der Langzeitstabilitäten der optoelektronischen Bauteile. Geeigneter sind sogenannte ambipolare Materialien, bei denen beide Funktionalitäten von ein und demselben Molekül erfüllt werden. Als lochtransportierende Einheiten (Donoren) der ambipolaren Moleküle werden vor allem Triarylamine oder Carbazol-Strukturen verwendet.

Der Erfindung lag die Aufgabe zu Grunde, verbesserte Verbindungen bereitzustellen, welche sich für den Einsatz in einer fluoreszierenden oder phosphoreszierenden oder einer thermisch aktivierten verzögerten Fluoreszenz (TADF) zeigenden OLED (TADF-OLED), insbesondere einer phosphoreszierenden oder TADF-OLED, eignen.

Die JP 2000 095766 A offenbart Benzoimidazol-Verbindungen sowie ihre Herstellung und Verwendung. Aus der WO 2012/056966 A1 sind aminenthaltende Phospinverbindungen bekannt. Die US 2014/343293 A1 offenbart 1,2,4-Triazol-basierte Derivate sowie deren Herstellung und Verwendung.

### Beschreibung

Die Erfindung stellt organische Moleküle der allgemeinen Formel 1 bereit, 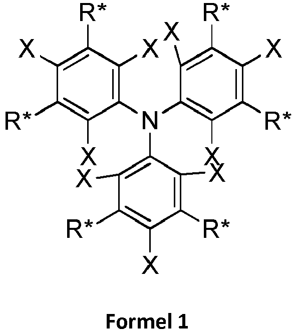 mit R* = Akzeptor-Rest A oder neutraler Rest R, der jeweils unabhängig voneinander über je eine der insgesamt 6 meta-Positionen des Triarylamins angebunden ist. Insgesamt ergibt die Zahl der Reste R und A genau 6, wobei die Zahl an Akzeptor-Resten A in Formel 1 mindestens 1 betragen muss.

R ist bei jedem Auftreten gleich oder verschieden Wasserstoff, Deuterium, Halogen, eine Alkylgruppe (unverzweigt, verzweigt oder zyklisch), eine Alkoxygruppe (unverzweigt, verzweigt oder zyklisch), eine Alkenylgruppe (unverzweigt, verzweigt oder zyklisch), oder eine Aryl- oder Heteroarylgruppe mit 5 bis 40 Ringatomen, die jeweils durch einen oder mehrere Reste R⁴ substituiert sein kann, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 40 aromatischen Ringatomen, die durch einen oder mehrere R⁴ substituiert sein kann, oder eine Aralkyl- oder Heteroaralkylgruppe mit 5 bis 40 aromatischen Ringatomen, die durch einen oder mehrere R⁴ substituiert sein kann, oder eine Kombination dieser Systeme; dabei können zwei oder mehrere Substituenten R auch miteinander, zusammen mit den Atomen, an die sie gebunden sind, ein mono- oder polycylisches aliphatisches oder aromatisches Ringsystem bilden;
R⁴ ist bei jedem Auftreten gleich oder verschieden H, D, OR⁵, SR⁵ oder C(R⁵)₃;
R⁵ ist bei jedem Auftreten gleich oder verschieden Wasserstoff, eine Alkylgruppe oder eine Arylgruppe;
und mit A = Akzeptorgruppe, unabhängig voneinander ausgewählt aus der Gruppe bestehend aus: 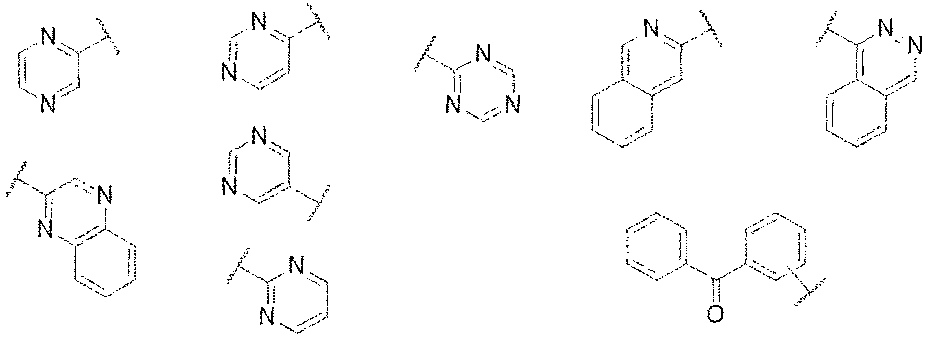 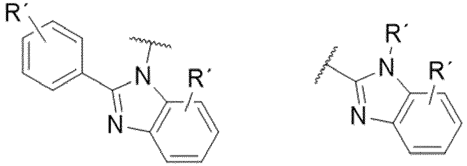 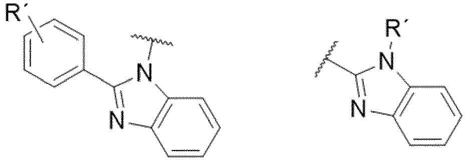 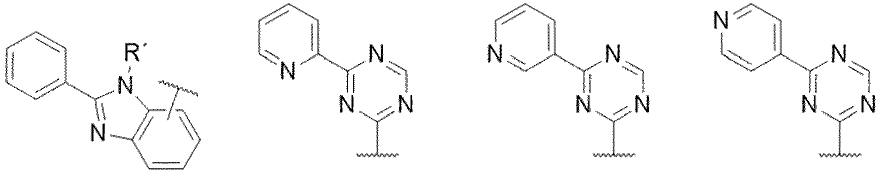 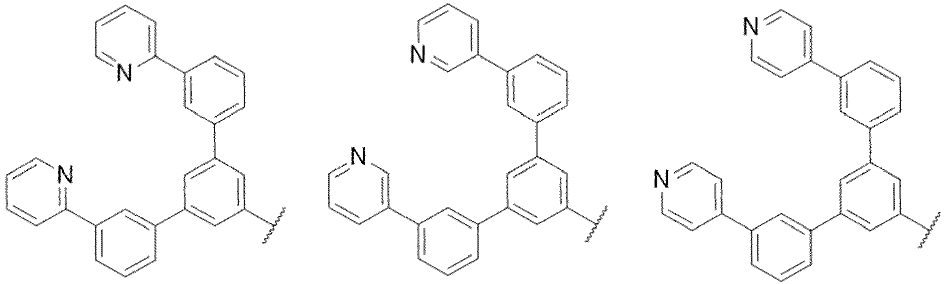 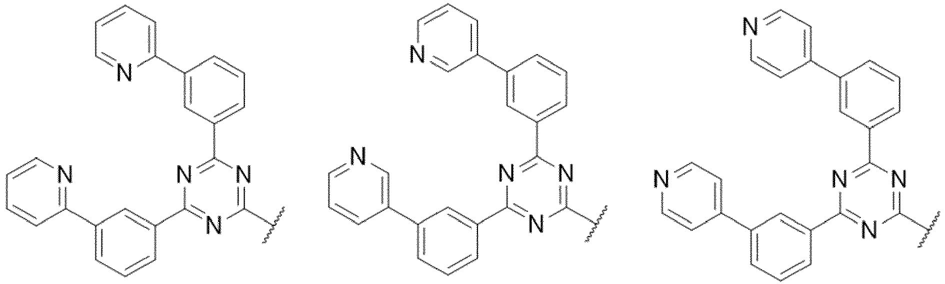 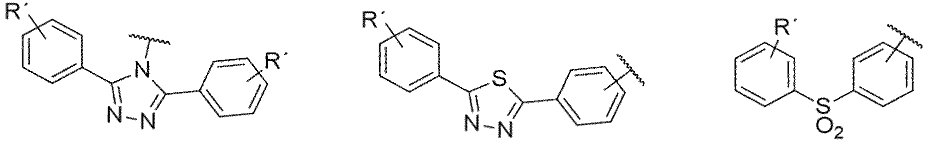 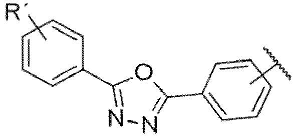 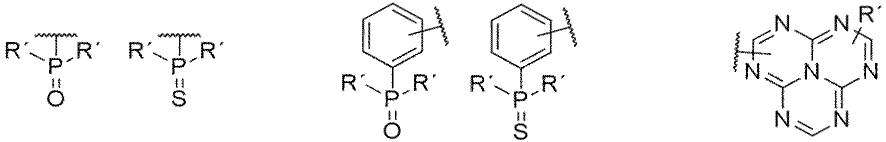 mit
gewellte Linie = Anbindungsposition für das Triarylamin;
Rest R' ist bei jedem Auftreten gleich oder verschieden Wasserstoff, Halogen, Deuterium, Gruppen, die über Stickstoff (-NR₂²) oder Sauerstoff- (-OR²) gebunden sind, Alkyl-(unverzweigt, verzweigt oder zyklisch), Heteroalkyl-, Aryl-, Heteroaryl-, Alkenyl-, Alkinyl-Gruppen oder substituierte Alkyl- (unverzweigt, verzweigt oder zyklisch), Heteroalkyl-, Aryl-, Heteroaryl- und Alkenyl-Gruppen, die mit Halogen oder Deuterium oder Alkylgruppen (unverzweigt, verzweigt oder zyklisch) substituiert sein können;
und mit X = unabhängig voneinander ein neutraler Rest R oder ein Aktivrest Z zur Einstellung der Grenzorbitalenergie der Triarylamineinheit;
und mit Z = Aktivrest, ausgewählt aus elektronenspendenen Gruppen, die über Stickstoff (-NR₂²) oder Sauerstoff- (-OR²) gebunden sind oder elektronenziehenden Gruppen wie Fluor, Nitril, (C=O)R²;
   R² ist bei jedem Auftreten gleich oder verschieden Wasserstoff, Deuterium, Alkyl-(unverzweigt, verzweigt oder zyklisch), Heteroalkyl-, Aryl-, Heteroaryl-, Alkenyl-, Alkinyl-Gruppen oder substituierte Alkyl- (unverzweigt, verzweigt oder zyklisch), Heteroalkyl-, Aryl-, Heteroaryl- und Alkenyl-Gruppen mit Substituenten wie Halogene oder Deuterium, Alkylgruppen (unverzweigt, verzweigt oder zyklisch);
In einer Ausführungsform ist A eine Akzeptorgruppe, unabhängig voneinander ausgewählt aus der Gruppe aus unsubstituiertem und substituiertem Pyridin, Triazin, Pyrimidin, P(=O)R¹₂, P(=O)R¹, P(=S)R¹₂, P(=S)R¹, P(=Se)R¹₂, P(=Se)R¹, Benzimidazol, 1,2,4-oxadiazol, 1,2,4-triazol, oder 1,2,3-Triazol; wobei die Verknüpfung vom Akzeptor zum Arylring desTriarylamins optional durch Substitution jedes H-Atoms des Akzeptors erfolgt.

In einer Ausführungsform handelt es sich bei den erfindungsgemäßen organischen Molekülen um Triarylamine der Formel 1 aufweisend mindestens einen Akzeptor-Rest A in meta-Position des Triarylamins, wobei der Akzeptor-Rest A einen Heteroaromaten mit einem N-Atom aufweist:

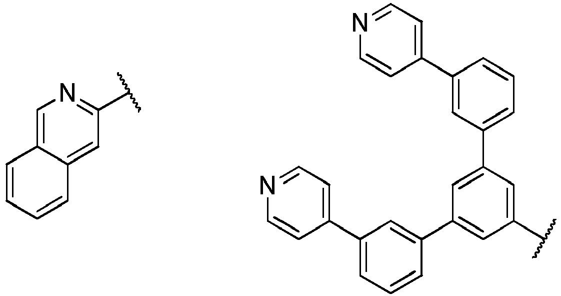

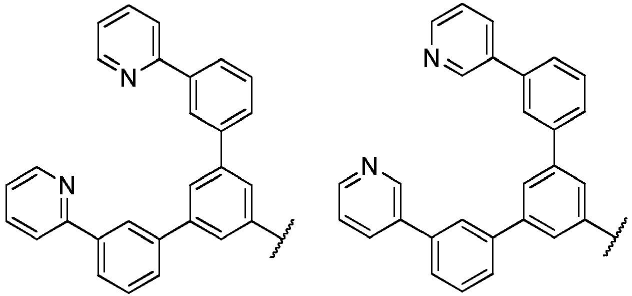

In einer weiteren Ausführungsform handelt es sich bei den erfindungsgemäßen organischen Molekülen um Triarylamine der Formel 1 aufweisend mindestens einen Akzeptor-Rest A in meta-Position des Triarylamins, wobei der Akzeptor-Rest A einen Heteroaromaten mit zwei N-Atomen aufweist:

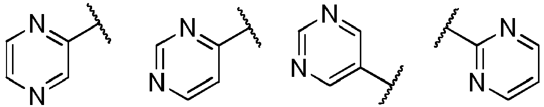

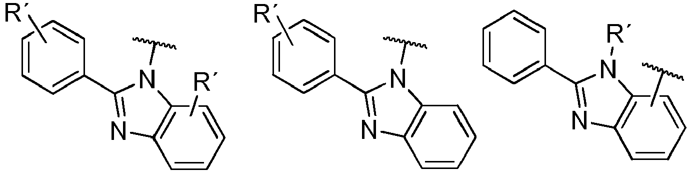

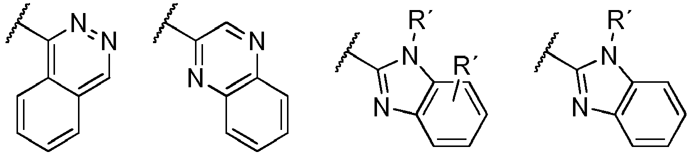

In einer Ausführungsform handelt es sich bei den erfindungsgemäßen organischen Molekülen um Triarylamine aufweisend mindestens einen Akzeptor-Rest A in meta-Position des Triarylamins, wobei der Akzeptor-Rest A einen Heteroaromaten mit mindestens drei N-Atomen und optional einen weiteren Heteroaromaten mit einem N-Atom aufweist:

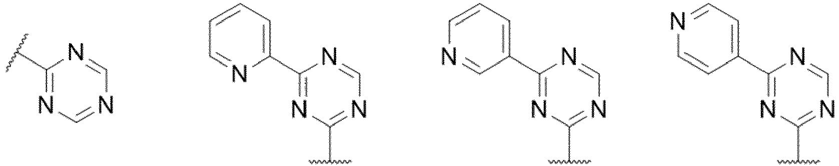

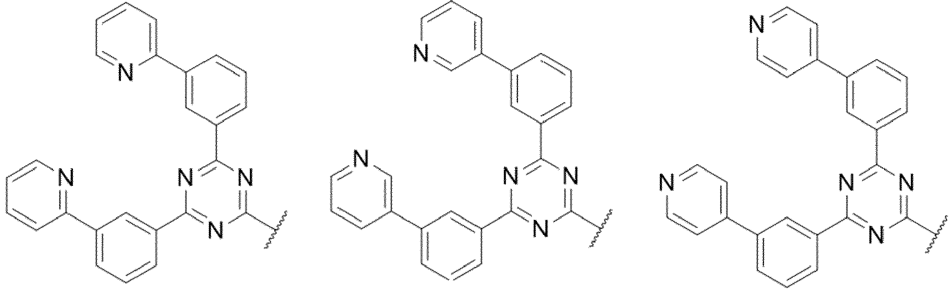

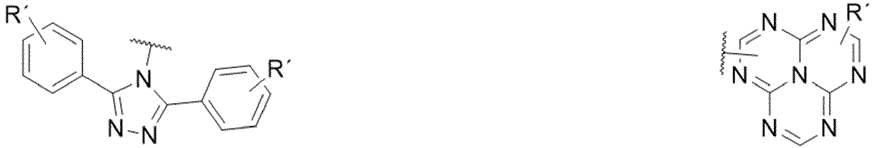

In einer weiteren Ausführungsform handelt es sich bei den erfindungsgemäßen organischen Molekülen um Triarylamine aufweisend mindestens einen Akzeptor-Rest A in meta-Position des Triarylamins, wobei der Akzeptor-Rest A S, O und/oder P und optional N enthält: 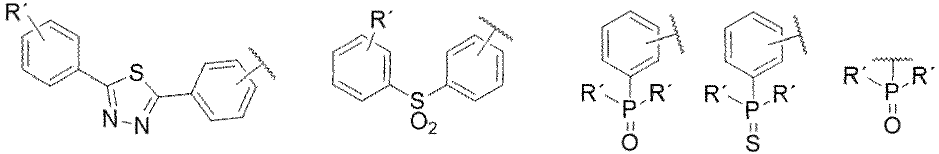 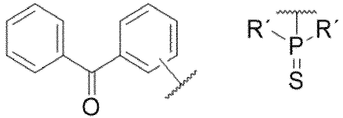 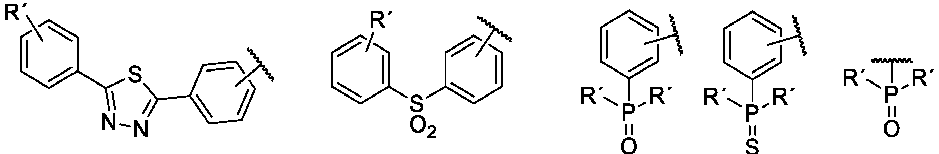 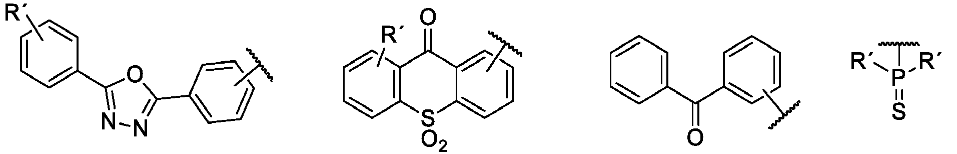 Überraschend wurde gefunden, dass die durch Formel 1 bestimmten Verbindungen zu deutlichen Verbesserungen der organischen Elektrolumineszenzvorrichtung führen, insbesondere hinsichtlich der Lebensdauer, der Effizienz und der Betriebsspannung. Dies gilt insbesondere auch für grün und blau phosphoreszierende Elektrolumineszenzvorrichtungen, vor allem bei Einsatz der erfindungsgemäßen Verbindungen als Matrixmaterial. Diese Materialien sowie organische Elektrolumineszenzvorrichtungen, welche derartige Verbindungen enthalten, sind daher der Gegenstand der vorliegenden Erfindung.

Durch Verknüpfung der in den Molekülen enthaltenen Donor- und Akzeptoreinheiten über die meta-Position des Triarylamin-Grundkörpers werden die Eigenschaften der Moleküle im Vergleich zu deren unverknüpften Derivaten signifikant verbessert, wodurch die Eignung für die Lösungsmittelbasierte Prozessierung stark erhöht wird, da einerseits die Löslichkeit ansteigt und andererseits die Tendenz zur Kristallisation nach oder während der Prozessierung, eines bei der Herstellung von OLEDs unerwünschten Vorgangs, effektiv unterbunden wird. Die Erfindung betrifft in einem Aspekt zuvor nicht beschriebene Moleküle. Hierbei werden durch geschickte Verknüpfung von Donor (elektronenreich) und Akzeptor (elektronenarm) ambipolare Moleküle geschaffen, die den notwendigen Kriterien eines Hostmoleküls in einem Wirt-Gast System als Emissionsschicht innerhalb einer OLED entsprechen und im Vergleich zu anders verknüpften, vergleichbaren Systemen energiereichere Emissionsfarben zugänglich machen, hier explizit auch Blau. Die erfindungsgemäßen organischen Moleküle weisen zudem eine hohe Triplett-Energie auf. Diese ist vor allem für die Kombination mit blauen Emittern vorteilhaft. Verursacht wird diese Eigenschaft durch die direkte Bindung von Donor- und Akzeptoreinheiten über die meta-Position des Triarylamin-Grundkörpers, ohne das z. B. ein Spacer vorhanden wäre. Bekannte Moleküle, bei denen die beiden Teile durch Brückeneinheiten miteinander verbunden sind, weisen deutlich geringere Triplett-Energien auf. Die Substituenten am Arylamin stellen das HOMO des Lochleiters ein (bis hin zu ca 6.2 eV). Die meta Verknüpfung zum Elektronenleiter sorgt für niedrige effektive Konjugation von Loch und Elektronenleiter (geringes ΔE_{S1,T1}, große Bandlücke), sodass die Verwendung in Kombination mit tiefblauen Emittern möglich wird.

Das elektronenreiche Segment besteht aus einem Triarylamin, das mit 1-6 elektronenarmen Molekülsegmenten wie z.B. Benzimidazol-, Phosphinoxid-, Triazolderivaten und/oder Kombinationen der jeweiligen elektronenarmen Verbindungen in meta Position zum Triarylamin Stickstoff verknüpft sind. Nun kann auf die elektrisch instabilen Carbazol-Derivate verzichtet werden, da durch geschickte Modifikation des Trialrylamins dessen reversible oxidierbarkeit gewährleistet ist, ohne auf tiefe HOMO Niveaus bei großer Bandlücke und hohen Tripletenergien verzichten zu müssen. Dies macht diese Materialien nicht nur für rote und grüne Emitter, sondern auch bis hin zu blauen Emittern zugänglich.

Ferner lassen sich durch die spezielle meta-Verknüpfung die Energieniveaus der Verbindungen gezielt und unabhängig voneinander einstellen. Dies erlaubt es, neben roten und grünen Emittern auch blaue Emitter zu verwenden, die in eine Matrix, die aus der erfindungsgemäßen Verbindung besteht, eingebettet werden. In einer optionalen Ausführung erfolgt die Einführung polymerisierbarer Gruppen. Diese Verbindungen werden homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020 , EP 894107 oder der EP 1669386), Paraphenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder der DE 102005037734) oder auch mehreren dieser Einheiten. Diese Polymere können auch noch weitere Einheiten enthalten, beispielsweise emittierende Einheiten, wie z. B. phosphoreszierende Metallkomplexe (z. B. gemäß WO 06/003000), und/oder Ladungstransporteinheiten, insbesondere solche basierend auf Triarylaminen. Die emittierenden Einheiten, beispielsweise die phosphoreszierenden Metallkomplexe, können auch zu dem Polymer zugemischt werden.

Optional werden in die erfindungsgemäßen Verbindungen Löslichkeitsvermittelnde Gruppen als Rest R eingeführt. Hierfür eignen sich beispielsweise Gruppen wie Alkyl, Alkoxy, verzweigte Alkyl- oder Alkoxyreste, Polyethylenglycole, sowie lineare oder verzweigte, fluorierte oder perfluorierte Alkylketten.

Optional werden an die Donor- und/oder Akzeptoreinheiten elektronenziehende oder - schiebende Gruppen (wie Halogene, Pseudohalogene, Alkyl, perfluoralkyl, Dialkylamino, Alkoxy, Carbonyle, Carboxyle) eingeführt, durch die sich das auf der Donor-Einheit lokalisierte HOMO sowie das auf der Akzeptoreinheit lokalisierte LUMO unabhängig voneinander einstellen lassen. Die Verwendung blau leuchtender Emitter wird ermöglicht.

Optional weisen die Reste R*, X und R' thermisch und/oder photoinduziert polymerisierbare Gruppen (auch unter Verwendung von Polymerisationsstartern) zur Polymeristaion im Film (Oxetan, Oxiran, Vinyl, Vinylether, Styrol, Acrylate, Zimtsäure) auf. Derartige funktionelle Gruppen sind dem Fachmann bekannt und werden beispielsweise bei Zuniga, C.; Barlow, S.; Marder, S. R. Chem. Mater. 2011, 23, 658, gelehrt.

Ein weiterer Aspekt der vorliegenden Erfindung betrifft die Verwendung eines organischen Moleküls der hierin beschriebenen Art in einer emittierenden Schicht als Matrixmaterial (Hostmaterial) und/oder Sensitizer für lumineszierende Emitter und/oder als Elektroneninjektionsmaterial und/oder als Elektronentransportmaterial und/oder als Lochinjektionsmaterial und/oder Lochtransportmaterial und/oder als Lochblockiermaterial und/oder als Elektronenblockiermaterial in einem optoelektronischen Bauelement.

In einer Ausführungsform der Erfindung wird das organische Molekül der hierin beschriebenen Art in einer emittierenden Schicht eines optoelektronischen Bauelementes, bevorzugt in Mischung mit mindestens einer weiteren Verbindung verwendet. Hierbei ist bevorzugt, dass das organische Molekül der hierin beschriebenen Art in der Mischung das Matrixmaterial ist.

In einem weiteren Aspekt der Erfindung wird ein organisches Moleküle der hierin beschriebenen Art entsprechend als Matrixmaterial für lumineszierende Emitter in einem optoelektronischen Bauelement eingesetzt.

Ein optoelektronisches Bauelement gemäß der vorliegenden Erfindung ist in einer Ausführungsform ausgewählt aus der Gruppe bestehend aus:
- Organischen Licht-emittierenden Bauteilen (OLEDs),
- Licht-emittierenden elektrochemischen Zellen,
- OLED-Sensoren, insbesondere in nicht hermetisch nach außen abgeschirmten Gas- und Dampf-Sensoren,
- organischen Solarzellen,
- Organischen Feldeffekttransistoren,
- Organischen Lasern und
- Down-Konversions-Elementen
- Up-Konversions-Elementen.

Ein weiterer Erfindungsaspekt betrifft Mischungen, enthaltend mindestens ein organisches Molekül der hierin beschriebenen Art und mindestens einen lumineszierenden Emitter.

In einem weiteren Aspekt betrifft die Erfindung Formulierungen enthaltend mindestens ein organisches Molekül der hierin beschriebenen Art oder eine hier beschriebene Mischung und mindestens ein Lösemittel.

In einer Ausführungsform beträgt der Anteil des organischen Materials als Matrixmaterial in optischen lichtemittierenden Bauelementen, insbesondere in OLEDs, zwischen 50.00 % und 99.99 %.

In einer weiteren Ausführungsform beträgt der Anteil des organischen Materials als CoHost oder Sensitizer, insbesondere in OLEDs, unter 1 %.

In einem weiteren Aspekt betrifft die Erfindung ein optoelektronisches Bauelement aufweisend ein organisches Molekül der hierin beschriebenen Art. Hierbei enthält ein erfindungsgemäßes optoelektronisches Bauelement mindestens eine Schicht zwischen Anode und Kathode, die ein organisches Molekül der hierin beschriebenen Art beispielsweise als Matrixmaterial (Hostmaterial), CoHost, Sensitizer für lumineszierende Emitter und/oder als Elektronentransportmaterial und/oder als Lochinjektionsmaterial und/oder als Lochblockiermaterial enthält. Hierbei kann das optoelektronische Bauelement ausgewählt sein aus der Gruppe bestehend aus organischem lichtemittierendem Bauelement, organischer Diode, organischer Solarzelle, organischem Transistor, organischer Licht-emittierender Diode, Licht-emittierender elektrochemischer Zelle, organischem Feldeffekttransistor und organischem Laser.

In einem weiteren Aspekt betrifft die Erfindung ein Verfahren zur Herstellung eines optoelektronischen Bauelements, wobei ein organisches Molekül der hierin beschriebenen Art verwendet wird.

Teil eines derartigen Verfahrens kann das Aufbringen eines erfindungsgemäßen organischen Moleküls auf einen Träger sein. Das Aufbringen kann optional durch Verdampfung im Vakuum oder nass-chemisch erfolgen.

Die hierfür notwendigen Verfahren sind dem Fachmann bekannt und können von ihm ohne weiteres auf optoelektronische Bauelemente enthaltend ein organisches Molekül der hierin beschriebenen Art angewandt werden.

Die erfindungsgemäßen organischen Moleküle weisen bei Verwendung in optoelektronischen Bauteilen folgende Vorteile gegenüber dem Stand der Technik auf: Die Ladungsträgermobilität im Bauteil ist verbessert, die Effizienz der Bauteile ist höher, die Stabilität der Bauteile ist höher und die Lebensdauer der Bauteile ist höher im Vergleich zu bekannten Systemen.

Durch die nachfolgenden Beispiele wird die Erfindung näher erläutert, ohne sie dadurch einzuschränken.

### Beispiele:

### Buchwald-Hartwig-Aminierung:

Alle Buchwald-Hartwig-Aminierungen wurden unter Schlenkbedingungen durchgeführt. Es wurden jeweils 1.0 eq Arylamin und 1.0 eq Bromaromat in trockenem Toluol (10 ml/mmol Amin) vorgelegt und mit 1.5 eq an Base pro reagierendes NH versetzt. Die zuvor angesetzten Katalysatorlösung, bestehend aus 0.01 eq Pd₂(dba)₃ und 0.016 eq P(*t*Bu)₃ in Toluol wurde anschließend zur Reaktionsmischung gegeben. Diese wurden 2-24 h bei 80 °C gerührt. Die Aufarbeitung erfolgte durch quenchen des Katalysators mit Wasser, worauf 3x mit Ethylacetat extrahiert wurde. Die vereinigten organischen Phasen wurden im Vakuum vom Lösemittel befreit und anschließend mittels Säulenchromatographie gereinigt (Eluent bestehend aus Cyclohexan / Ethylacetat Mischungen, graduiert).

### Allgemeine Syntheseroute zu Ambipolaren Hosts der Familie 1

### Stufe 1, Synthese des funktionalisierten Akzeptorgerüstes:

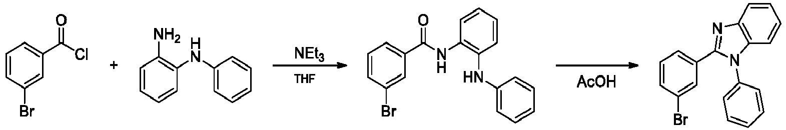

In einem ausgeheizten Schlenkkolben wurden 8.57 g (98%) N-Phenyl-o-phenylendiamin (45.6 mmol, 1.0 eq) in 100 ml trockenem THF unter N2 Atmosphäre vorgelegt, worauf langsam 10.00 g 3-Bromobenzoylchlorid (45.6 mmol, 1.0 eq) vorsichtig zugetropft wurden. Letztlich wurden 4.64 g Triethylamin (45.6 mmol, 1.0 eq) vorsichtig zugetropft und bei Raumtemperatur für 24 h gerührt. Darauf wurde die Reaktionslösung mit Wasser verdünnt und 3x mit Ethylacetat extrahiert. Die vereinigten organischen Phasen wurden über MgSO₄ getrocknet und vom Lösemittel befreit. Der erhaltene Feststoff wurde anschließend in 200 ml Essigsäure für 5 h refluxiert. Anschließend wurde die Essigsäure im Vakuum entfernt. Das Rohprodukt wurde anschließend säulenchromatographisch (Cyclohexan:Ethylacetat = 9:1) gereinigt. Es wurden 14.135 g, 89 % d. Theorie erhalten.

### Stufe 2: Synthese der Donor-Vorstufe:

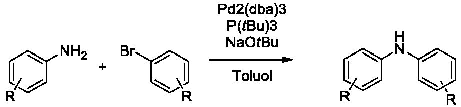

Die verschiedenen Diarylamine wurden nach der allgemeinen Synthesevorschrift für Buchwald Hartwig Aminierungen durchgeführt. Die Ausbeuten betrugen zwischen 57 % und 93 %

### Stufe 3: Zusammenführung der Vorstufen mittels Buchwald-Hartwig-Aminierung:

Die verschiedenen Triarylamine wurden nach der allgemeinen Synthesevorschrift für Buchwald Hartwig Aminierungen durchgeführt. Die Ausbeuten betrugen zwischen 88 % und 94 %

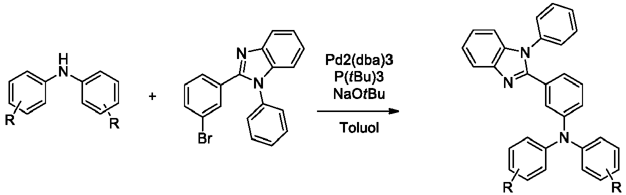

### Beispiel 1: Molekül 1

Es wurden 1500 mg (4.3 mmol, 1.0 eq) des Akzeptorgerüstes und 986 mg (4.3 mmol, 1.0 eq) des Diarylamins mittels allgemeiner Buchwald-Hartwig-Aminierung zur Reaktion gebracht. Es wurden 1.999 g, 93 % Produkt erhalten.

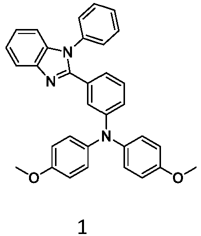

### Beispiel 2: Molekül 2

Es wurden 1500 mg (4.3 mmol, 1.0 eq) des Akzeptorgerüstes und 848 mg (4.3 mmol, 1.0 eq) des Diarylamins mittels allgemeiner Buchwald-Hartwig-Aminierung zur Reaktion gebracht. Es wurden 1.874 g, 94 % Produkt erhalten.

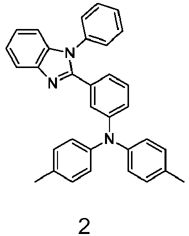

### Beispiel 3: Molekül 3

Es wurden 1000 mg (2.86 mmol, 1.0 eq) des Akzeptorgerüstes und 965 mg (2.86 mmol, 1.0 eq) des Diarylamins mittels allgemeiner Buchwald-Hartwig-Aminierung zur Reaktion gebracht. Es wurden 1.531 g. 88 % Produkt erhalten.

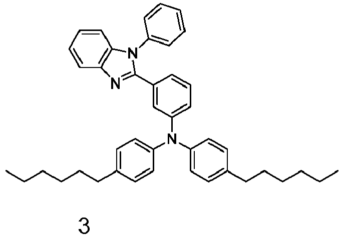

### Beispiel 4: Molekül 4

Es wurden 2000 mg (5.73 mmol, 1.0 eq) des Akzeptorgerüstes und 1175 mg (5.73 mmol, 1.0 eq) des Diarylamins mittels allgemeiner Buchwald-Hartwig-Aminierung zur Reaktion gebracht. Es wurden 2.395 g, 88 % Produkt erhalten.

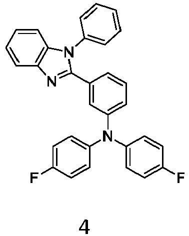

### Beispiel 5: Molekül 5

Es wurden 800 mg (1.83 mmol, 1.0 eq) des Akzeptorgerüstes und 507 mg (1.83 mmol, 1.0 eq) des Diarylamins mittels allgemeiner Buchwald-Hartwig-Aminierung zur Reaktion gebracht. Es wurden 0.917 g, 92 % Produkt erhalten.

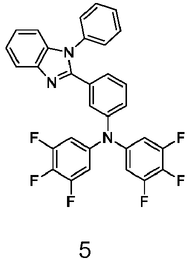

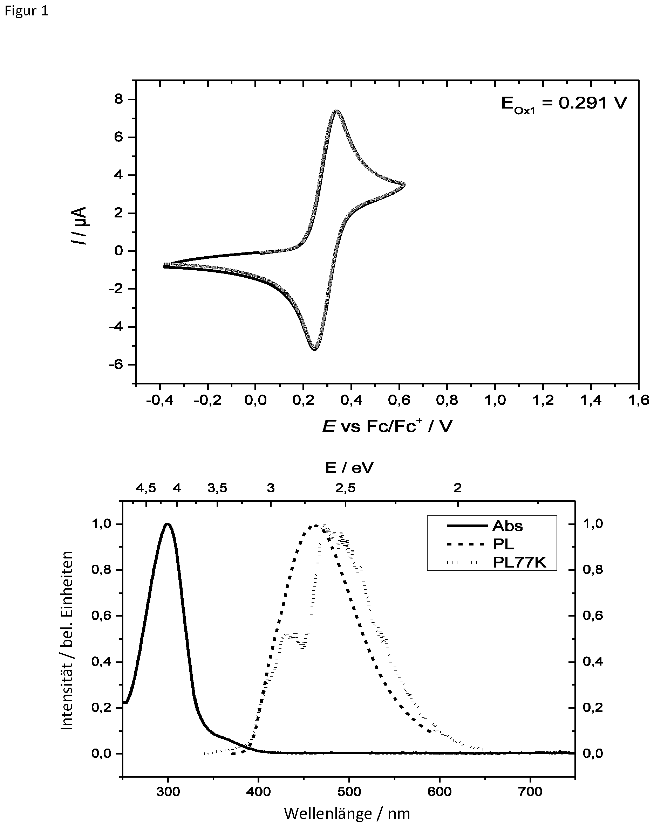

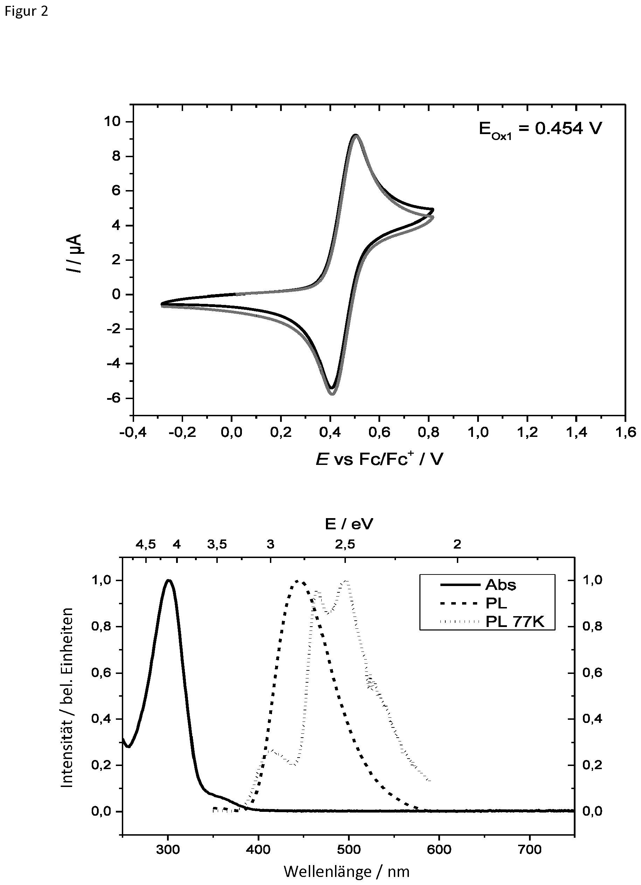

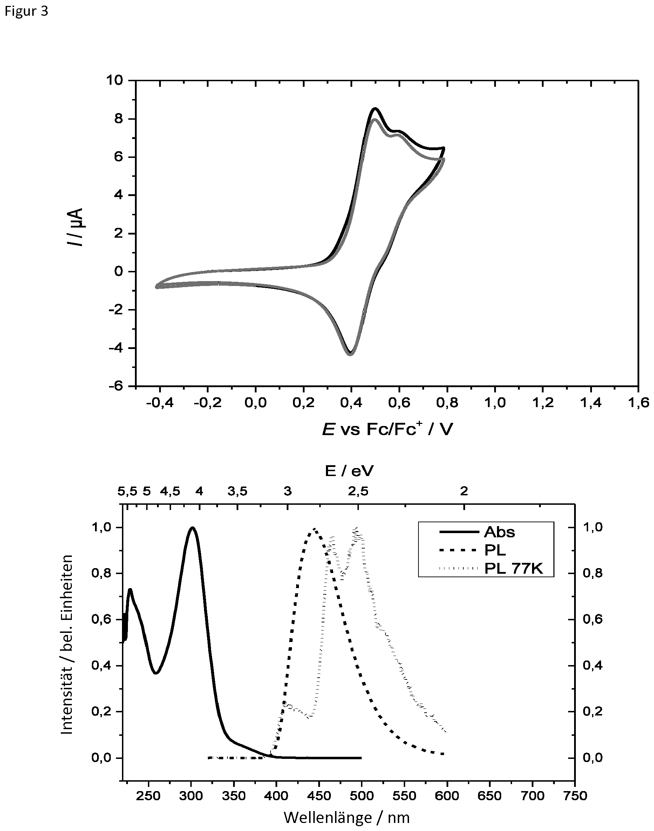

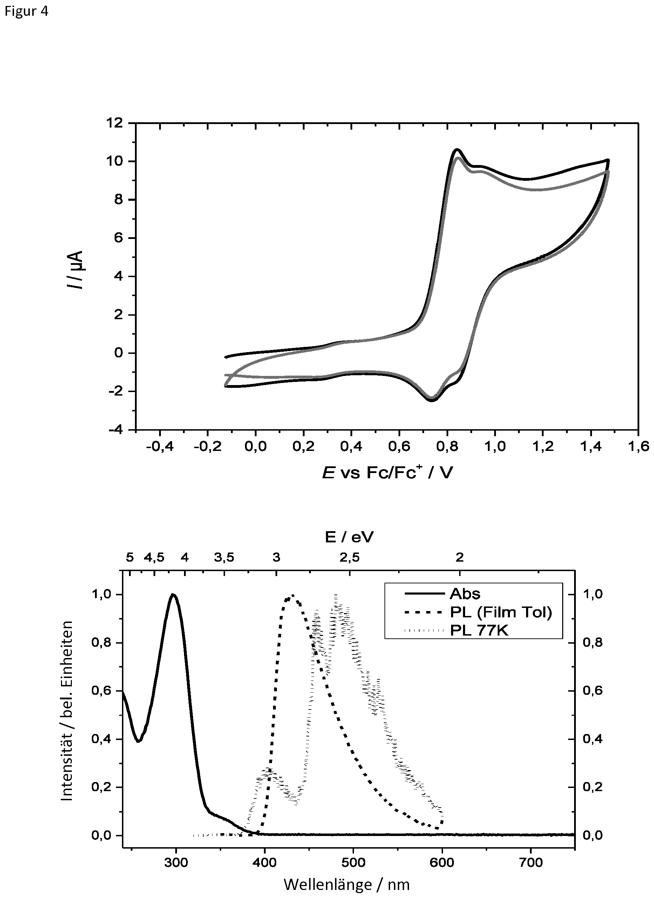

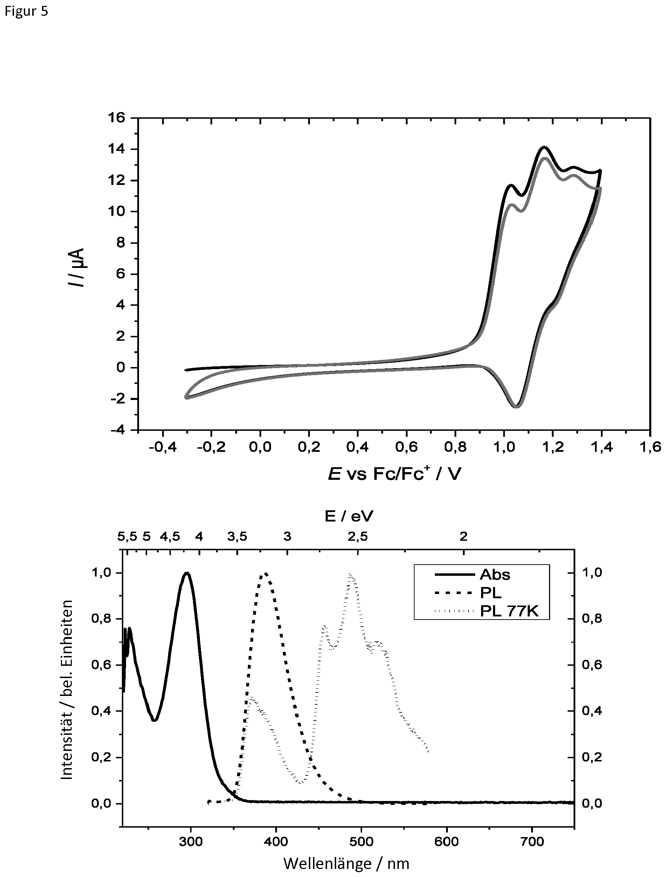

In den Figuren 1 bis 5 sind die elektrochemischen sowie photophysikalischen Messergebnisse für die Moleküle 1 bis 5 dargestellt.

Beispielhaft werden die aus den elektrochemischen sowie photophysikalischen Messungen extrahierten Werte tabellarisch aufgeführt:

**Tab 1: Es werden das Oxidationspotential gegen Ferrocene gemessen in Volt (E_{Ox} vs Fc / V), die bathochrome Absorptionskante in Nanometer (Abs Icutoff / nm), das Absorptionsmaximum in Nanometer (Abs Imax / nm), die errechnete HOMO Energie in Elektronenvolt (E_{HOMO} / eV), die errechnete LUMO Energie in Elektronenvolt (E_{LUMO} / eV), der S1-Zustand in Elektronenvolt aus der Photolumineszenz bei maximaler Intensität (PLₘₐₓ S1 / eV), der T1-Zustand in Elektronenvolt aus der tieftemperatur Photolumineszenz bei maximaler Intensität (PLₘₐₓ T1 / eV), die Energiedifferenz zwischen S1 und T1 Zustand aus der Photolumineszenz bei maximaler Intensität (DE_{ST, max} / eV), der S1-Zustand in Elektronenvolt aus der hypsochromen Emissionskante der Photolumineszenz (PLₒₙₛₑₜ S1 / eV), der T1-Zustand in Elektronenvolt aus der hypsochromen Emissionskante der tieftemperatur Photolumineszenz (PLₒₙₛₑₜ T1 / eV), die Energiedifferenz zwischen S1 und T1 Zustand aus den hypsochromen Emissionskanten der Photolumineszenz (DE_{ST, max} / eV). Epa ist das anodische Peakpotential der entsprechenden cyclovoltammetrischen Messung, ir steht für einen irreversiblen Elektronentransfer, r für einen reversiblen Elektronentransfer.**

| Material | E_{Ox} vs Fc/V | Abs λ_{cutoff} / nm | Abs λₘₐₓ / nm | E_{HOMO}/ eV | E_{LUMO} / eV | PLₘₐₓ S₁/eV | PLₘₐₓ T₁/eV | ΔE_{ST}, max / eV | PLₒₙₛₑₜ S₁/eV | PLₒₙₛₑₜ T₁/eV | ΔE_{ST,onset} / eV |
|---|---|---|---|---|---|---|---|---|---|---|---|
| 1 | 0.29 | 336 | 299 | -5.39 | -1.70 | 2,68 | 2,62 | 0.06 | 3,19 | 2,84 | 0,35 |
| 2 | 0.45 | 335 | 301 | -5.55 | -1.85 | 2.78 | 2.66 | 0.12 | 3,10 | 2,80 | 0,30 |
| 3 | 0.45 | 336 | 301 | -5.55 | -1.80 | 2.79 | 2.66 | 0.13 | 3,05 | 2,80 | 0,25 |
| 4 | 0.79 | 332 | 297 | -5.89 | -2,15 | 2.88 | 2.70 | 0.18 | 3,09 | 2,80 | 0,29 |
| 5 | 1.03 | 330 | 295 | -6.08 | -2,33 | 3.21 | 2.71 | 0.50 | 3,50 | 2,85 | 0,65 |
| | Epa (ir) | | | -6.21 | | | | | | | |
| | 1.11 (r) | | | | | | | | | | |

### Allgemeine Syntheseroute zu ambipolaren Hosts der Familie 2

Zur Synthese der Hostmaterialien der Familie 2 wurde zur Generierung der Donorkomponente zwei Äquivalente des funktionalisierte Akzeptors mit einem Äquivalent des entsprechenden Anilinderivats mittel Buchwald-Hartwig Aminierung zur Reaktion gebracht.

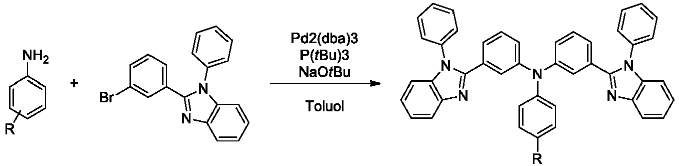

### Beispiel 6: Molekül 6:

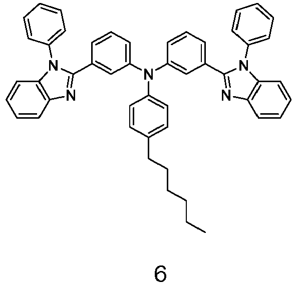

Es wurden 2000 mg (5.73 mmol, 2.0 eq) des Akzeptorgerüstes und 508 mg (1.83 mmol, 1.0 eq) des Diarylamins mittels allgemeiner Buchwald-Hartwig-Aminierung zur Reaktion gebracht. Es wurden 1.690 g, 83 % Produkt erhalten.

### Beispiel 7: Molekül 7

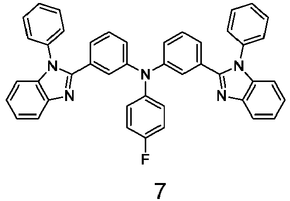

Es wurden 2000 mg (5.73 mmol, 2.0 eq) des Akzeptorgerüstes und 322 mg (1.83 mmol, 1.0 eq) des Diarylamins mittels allgemeiner Buchwald-Hartwig-Aminierung zur Reaktion gebracht. Es wurden 900 mg, 48 % Produkt erhalten.

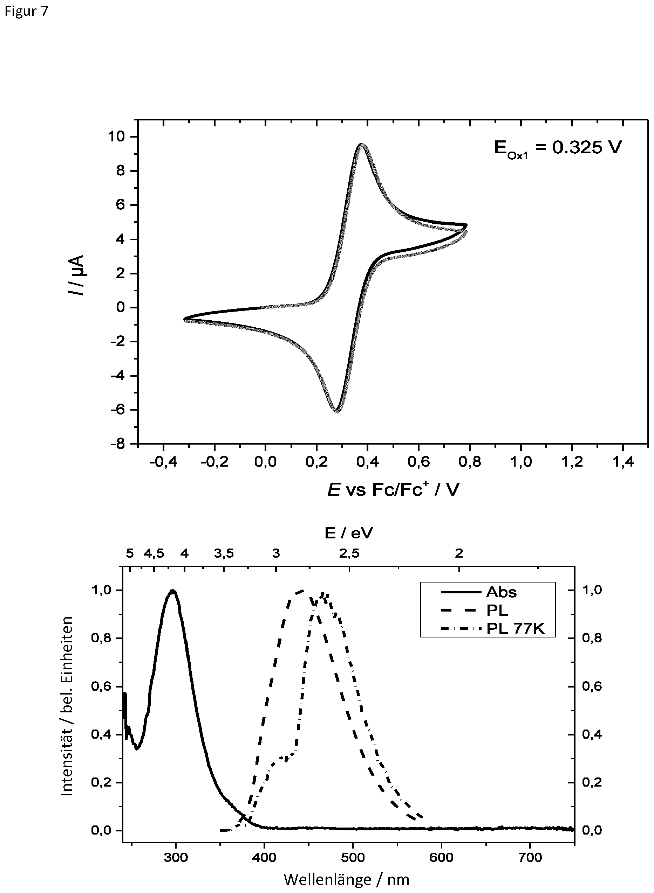

In Figur 7 sind die elektrochemischen sowie photophysikalischen Messergebnisse für das Molekül 6 dargestellt.

Beispielhaft werden die aus den elektrochemischen sowie photophysikalischen Messungen extrahierten Werte tabellarisch aufgeführt:

**Tab 2: Es werden das Oxidationspotential gegen Ferrocene gemessen in Volt (E_{Ox} vs Fc / V), die bathochrome Absorptionskante in Nanometer (Abs Icutoff / nm), das Absorptionsmaximum in Nanometer (Abs Imax / nm), die errechnete HOMO Energie in Elektronenvolt (E_{HOMO} / eV), die errechnete LUMO Energie in Elektronenvolt (E_{LUMO} / eV), der S1-Zustand in Elektronenvolt aus der Photolumineszenz bei maximaler Intensität (PLₘₐₓ S1 / eV), der T1-Zustand in Elektronenvolt aus der tieftemperatur Photolumineszenz bei maximaler Intensität (PLₘₐₓ T1 / eV), die Energiedifferenz zwischen S1 und T1 Zustand aus der Photolumineszenz bei maximaler Intensität (DE_{ST, max} / eV), der S1-Zustand in Elektronenvolt aus der hypsochromen Emissionskante der Photolumineszenz (PLₒₙₛₑₜ S1 / eV), der T1-Zustand in Elektronenvolt aus der hypsochromen Emissionskante der tieftemperatur Photolumineszenz (PLₒₙₛₑₜ T1 / eV), die Energiedifferenz zwischen S1 und T1 Zustand aus den hypsochromen Emissionskanten der Photolumineszenz (DE_{ST, max} / eV). Epa ist das anodische Peakpotential der entsprechenden cyclovoltammetrischen Messung, ir steht für einen irreversiblen Elektronentransfer, r für einen reversiblen Elektronentransfer.**

| Material | E_{Ox} vs Fc / V | Abs λ_{cutoff} / nm | Abs λₘₐₓ / nm | E_{HOMO}/ eV | E_{LUMO} / eV | PLₘₐₓ S₁/eV | PLₘₐₓ T₁ / eV | ΔE_{ST} / eV | PLₒₙₛₑₜ S₁ / eV | P Lₒₙₛₑₜ T₁ / eV | ΔE_{ST, onset} / eV |
|---|---|---|---|---|---|---|---|---|---|---|---|
| 6 | 0.58 | 394 | 302 | -5.68 | -2.53 to -2.00 | 2.93 | 2.69 | 0.24 | 3,20 | 2,81 | 0,49 |

### Allgemeine Synthesevorschrift des funktionalisierten Akzeptors für ambipolare Hosts der Familie 3

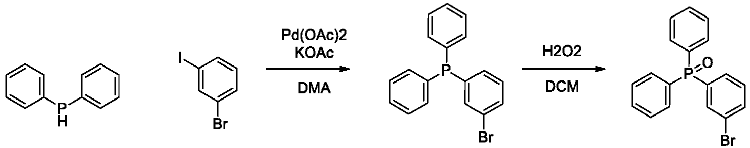

Es wurden 4.380 g (23.3 mmol, 1.0 eq) des Diphenylphosphins, 6.726 g des 1-Bromo-3-lodobenzols (23.3 mmol, 1.0 eq) und 2.512 g (25.6 mmol, 1.1 eq) Kaliumacetat in 30 ml N,N'-Dimethylacetamid unter Stickstoffatmosphäre vorgelegt und mit 0.026 g (0.117 mmol, 0.005 eq) Palladium(II)acetat versetzt. Die Reaktionsmischung wurde für 3 Stunden bei 130 °C gerührt und vom Lösemittel befreit. Der so erhaltene Feststoff (6.890 g) wurde in 50 ml Dichlormethan gelöst und mit ca 10 Äquivalenten Wasserstoffperoxid (6.870 g, 202 mmol, 10 eq) versetzt. Die Reaktionsmischung wurde 3 Stunden bei Raumtemperatur gerührt. Anschließend wurde die Reaktionsmischung mit Wasser verdünnt, dreifach mit Dichlormethan extrahiert, über Magnesiumsulfat getrocknet und im Vakuum vom Lösemittel befreit. Der so erhaltene Feststoff wurde zweich unter Verwendung von Gradienten von Dichlormethan Ethylacetat Eluenten säulenchromatographisch aufgereinigt Es wurden 6.927 g (20.2 mmol) 87 % in Bezug auf das Diphenylphospin enthalten.

Die verschiedenen Triarylamine der Familie 3 wurden nach der allgemeinen Synthesevorschrift für Buchwald Hartwig Aminierungen durchgeführt. Die Ausbeuten betrugen zwischen 88 % und 94 %

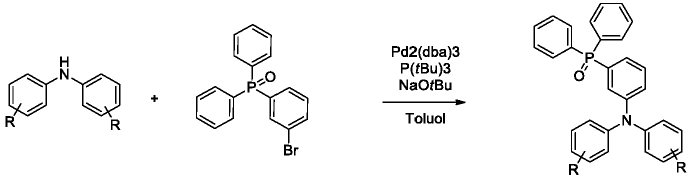

### Beispiel 8: Molekül 8

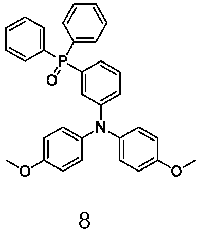

Es wurden 1400 mg (3.92 mmol, 1.0 eq) des Akzeptorgerüstes und 899 mg (3.92 mmol, 1.0 eq) des Diarylamins mittels allgemeiner Buchwald-Hartwig-Aminierung zur Reaktion gebracht. Es wurden 1.530 g. 77 % Produkt erhalten.

### Beispiel 9: Molekül 9

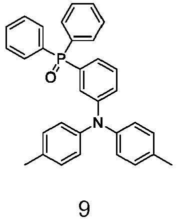

Es wurden 1400 mg (3.92 mmol, 1.0 eq) des Akzeptorgerüstes und 773 mg (3.92 mmol, 1.0 eq) des Diarylamins mittels allgemeiner Buchwald-Hartwig-Aminierung zur Reaktion gebracht. Es wurden 1.377 g. 74 % Produkt erhalten.

### Beispiel 10: Molekül 10

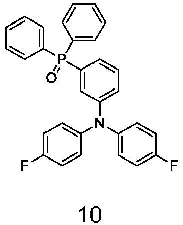

Es wurden 2000 mg (5.60 mmol, 1.0 eq) des Akzeptorgerüstes und 1260 mg (6.16 mmol, 1.1 eq) des Diarylamins mittels allgemeiner Buchwald-Hartwig-Aminierung zur Reaktion gebracht. Es wurden 1.960 g, 73 % Produkt erhalten.

### Beispiel 11: Molekül 11

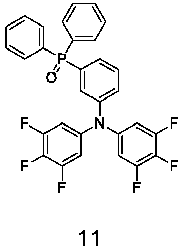

Es wurden 629 mg (1.76 mmol, 1.0 eq) des Akzeptorgerüstes und 488 mg (1.76 mmol, 1.0 eq) des Diarylamins mittels allgemeiner Buchwald-Hartwig-Aminierung zur Reaktion gebracht. Es wurden 470 mg, 73 % Produkt erhalten.

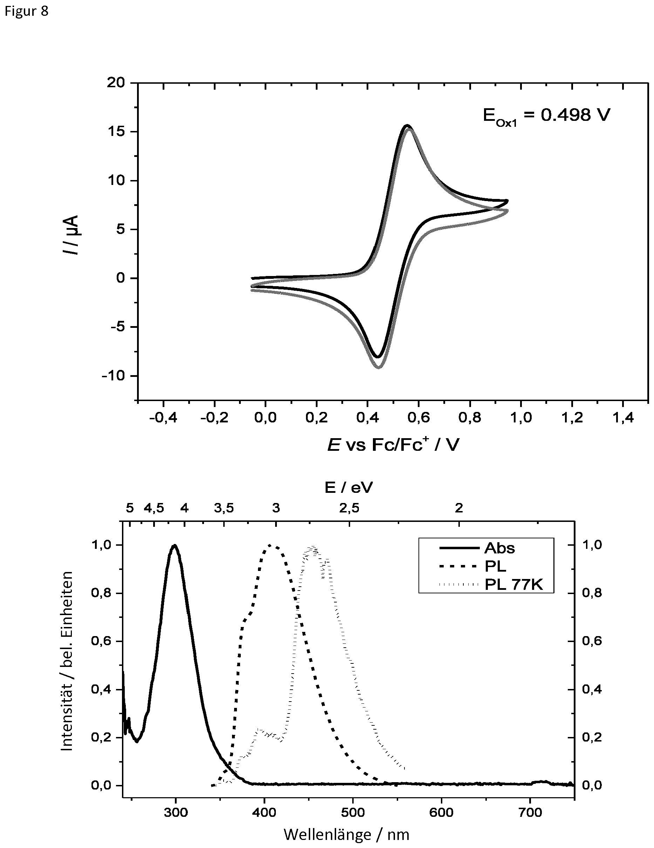

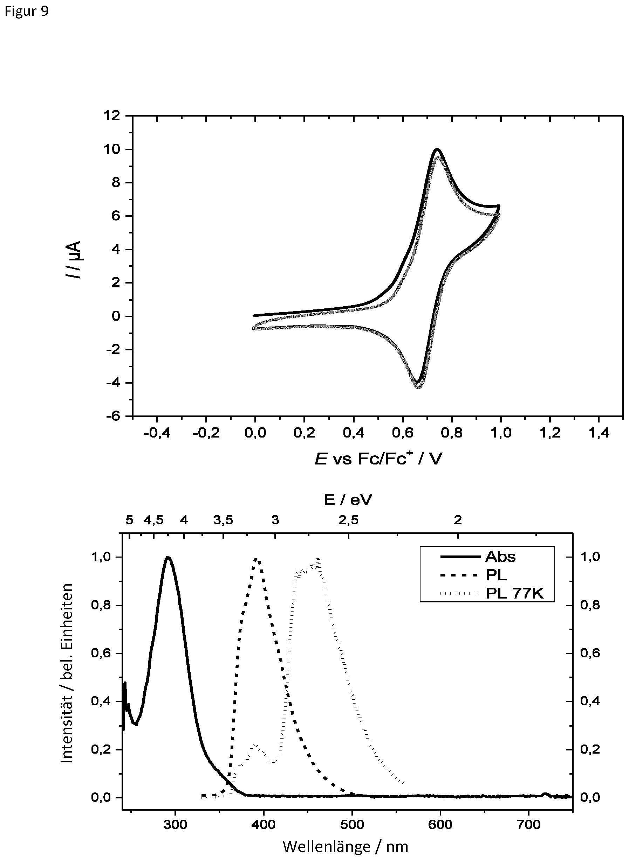

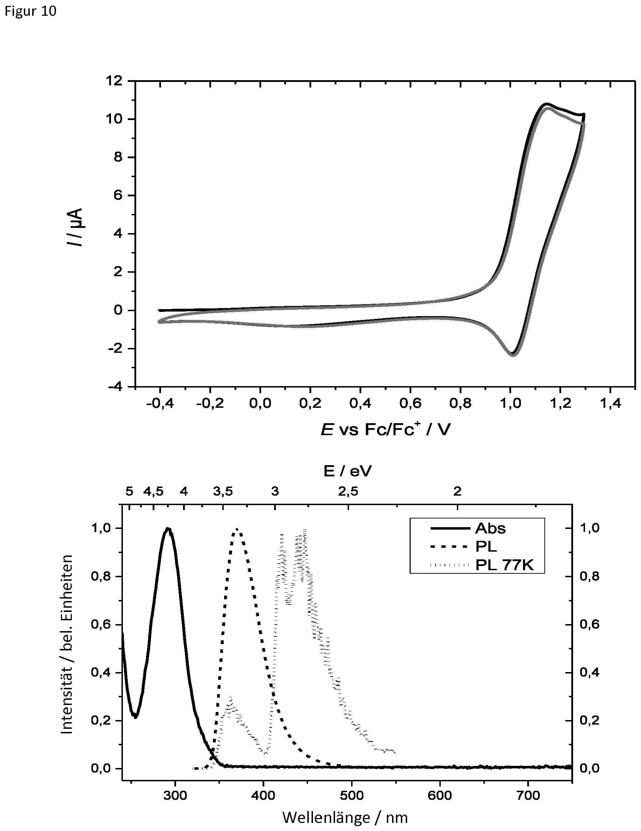

In den Figuren 7 bis 10 sind die elektrochemischen sowie photophysikalischen Messergebnisse für die Moleküle 8 bis 11 dargestellt.

**Tab 3: Es werden das Oxidationspotential gegen Ferrocene gemessen in Volt (E_{Ox} vs Fc / V), die bathochrome Absorptionskante in Nanometer (Abs Icutoff / nm), das Absorptionsmaximum in Nanometer (Abs Imax / nm), die errechnete HOMO Energie in Elektronenvolt (E_{HOMO} / eV), die errechnete LUMO Energie in Elektronenvolt (E_{LUMO} / eV), der S1-Zustand in Elektronenvolt aus der Photolumineszenz bei maximaler Intensität (PLₘₐₓ S1 / eV), der T1-Zustand in Elektronenvolt aus der tieftemperatur Photolumineszenz bei maximaler Intensität (PLₘₐₓ T1 / eV), die Energiedifferenz zwischen S1 und T1 Zustand aus der Photolumineszenz bei maximaler Intensität (DE_{ST, max} / eV), der S1-Zustand in Elektronenvolt aus der hypsochromen Emissionskante der Photolumineszenz (PLₒₙₛₑₜ S1 / eV), der T1-Zustand in Elektronenvolt aus der hypsochromen Emissionskante der tieftemperatur Photolumineszenz (PLₒₙₛₑₜ T1 / eV), die Energiedifferenz zwischen S1 und T1 Zustand aus den hypsochromen Emissionskanten der Photolumineszenz (DE_{ST, max} / eV). Epa ist das anodische Peakpotential der entsprechenden cyclovoltammetrischen Messung, ir steht für einen irreversiblen Elektronentransfer, r für einen reversiblen Elektronentransfer.**

| Material | E_{Ox} vs Fc / V | Abs λ_{cutoff} / nm | Abs λₘₐₓ / nm | E_{HOMO}/ eV | E_{LUMO} / eV | PLₘₐₓ S₁ / eV | PLₘₐₓ T₁ / eV | ΔE_{ST} / eV | PLₒₙₛₑₜ S₁ / eV | PLₒₙₛₑₜ T₁ / eV | ΔE_{ST} / eV |
|---|---|---|---|---|---|---|---|---|---|---|---|
| 8 | 0.33 | 344 | 297 | -5.43 | -1.83 | 2.80 | 2.66 | 0.14 | 3,28 | 2,90 | 0,38 |
| 9 | 0.50 | 344 | 298 | -5,60 | -2.00 | 3.03 | 2.75 | 0.28 | 3,43 | 2,95 | 0,48 |
| 10 | 0.70 | 335 | 291 | -5.80 | -2.10 | 3.15 | 2.80 | 0.35 | 3,45 | 2,99 | 0,46 |
| 11 | 1.08 | 328 | 291 | -6.18 | -2.38 | 3.35 | 2.95 | 0.40 | 3,62 | 3,06 | 0,68 |

### Allgemeine Syntheseroute zu ambipolaren Hosts der Familie 4

### Stufe 1, Synthese des funktionalisierten Akzeptorgerüstes:

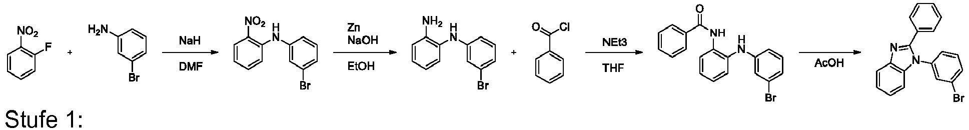

In einem ausgeheizten Schlenkkolben wurden 2.690 g 3-Bromoanilin (15.6 mmol, 1.1 eq) in 50 ml Dimethylformamid vorgelegt und unter Stickstoffstrom portionsweise mit 0.852 g (21.3 mmol, 1.5 eq) Natriumhydrid versetzt. Nach ca. einer Stunde war die dabei eintretende Gasentwicklung abgeschlossen, worauf 2.690 g 1-Fluor-2-Nirtrobenzol (14.2 mmol, 1.0 eq) zugegeben wurden. Der Schlenkkolben wurde mit einem Rückflusskühler versehen worauf das Lösemittel 3fach entgast wurde. Darauf wurde 12 h bei 140 °C gerührt. Darauf wurde das Lösemittel weitestgehend aus dem Reaktionsgefäß im Unterdruck entfernt. Die zurückbleibende Reaktionslösung wurde in Wasser gegeben und 5fach mit Dichlormethan extrahiert. Die vereinigten organischen Phasen wurden über Magnesiumsulfat getrocknet, filtriert und im Vakuum vom Lösemittel befreit. Die Aufreinigung erfolgte säulenchromatographisch. Es wurden 1170 mg, 28 % des Produktes erhalten.

### Stufe 2:

Das aus Stufe 1 erhaltene (*m*-Bromophenyl)(o-nitrophenyl)amin wurde in einer Menge von 1100 mg (3.75 mmol, 1.0 eq) in einem Reaktionsgefäß vorgelegt und in der Wärme in Ethanol gelöst. Darauf wurden 1130 mg Natronplätzchen in 2,5 ml Wasser gelöst und zur Reaktionsmischung gegeben. Anschließend wurden 3680 mg (56.2 mmol, 15 eq) Zinkstaub zugegeben und ca 8 Stunden bei 80 °C gerührt. Die Reaktionslösung wurde anschließend filtriert, das Filtrat wurde mit Ethylacetat gespült und mit wässriger Natriumhydrogencarbonatlösung extrahiert. Die organische Phase wurde über Magnesiumsulfat getrocknet, filtriert und im Vakuum vom Lösemittel befreit. Das Rohprodukt wurde säulenchromatographisch gereinigt. Es wurden 770 mg, 78 % der Theorie erhalten.

### Stufe 3:

Es wurden 700 mg *N*-3-Bromophenyl-*o*-phenylenediamin (2.66 mmol, 1.0 eq) in einem ausgeheizten Schlenkkolben in 40 ml trockenem THF unter Stickstoffatmosphäre vorgelegt. Darauf wurden 374 mg Benzoylchlorid (2.66 mmol, 1.0 eq) zugegeben, worauf 271 mg Triethylamin (2.66 mmol, 1.0 eq) langsam zugetropft wurden. Die Reaktionslösung wurde 24 h bei Raumtemperatur gerührt. Anschließend wurde die Reaktionslösung mit Wasser versetzt und 3fach mit Ethylacetat extrahiert. Die vereinigten organischen Phasen wurden über Magnesiumsulfat getrocknet, filtriert und im Vakuum vom Lösemittel befreit. Die Reinigung erfolgte säulenchromatographisch. Es wurden 970 mg, 99 % der Theorie, erhalten.

### Stufe 4:

Die 970 mg aus Stufe 3 wurden in einem 250 ml Rundkolben in ca 200 ml Essigsäure gelöst und 5 h refluxiert. Anschließend wurde die Essigsäure im Vakuum entfernt, das so erhaltene Rohprodukt wurde säulenchromatographisch aufgereinigt. Es ergaben sich 810 mg, 89 % der Theorie, Produkt.

Die verschiedenen Triarylamine der Familie 4 wurden nach der allgmeinen Synthesevorschrift für Buchwald Hartwig Aminierungen durchgeführt. Die Ausbeuten betrugen zwischen 12 % und 92 %

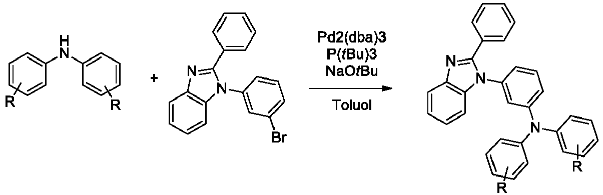

### Beispiel 12: Molekül 12:

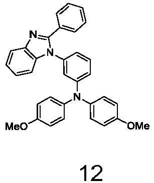

Es wurden 594 mg (1.7 mmol, 1.0 eq) des 1-(3-bromophenyl)-2-phenyl-1H-benzimidazols mit 390 mg (1.7 mmol, 1.0 eq) des entsprechenden Diarylamins versetzt und 4 h bei 80 °C in 50 ml trockenem Toluol umgesetzt. Nach säulenchromatographischer Aufarbeitung ergaben sich 492 mg Produkt, 58 % der Theorie.

### Beispiel 13: Molekül 13

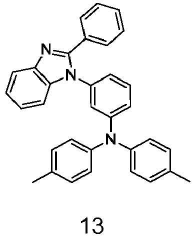

Es wurden 594 mg (1.7 mmol, 1.0 eq) des 1-(3-bromophenyl)-2-phenyl-1H-benzimidazols mit 335 mg (1.7 mmol, 1.0 eq) des entsprechenden Diarylamins versetzt und 4 h bei 80 °C in 50 ml trockenem Toluol umgesetzt. Nach säulenchromatographischer Aufarbeitung ergaben sich 95 mg Produkt, 12 % der Theorie.

### Beispiel 14: Molekül 14

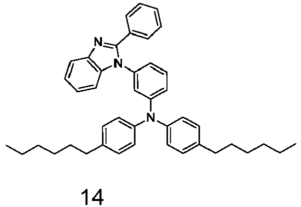

Es wurden 615 mg (1.76 mmol, 1.0 eq) des 1-(3-bromophenyl)-2-phenyl-1H-benzimidazols mit 594 mg (1.7 mmol, 1.0 eq) des entsprechenden Diarylamins versetzt und 4 h bei 80 °C in 50 ml trockenem Toluol umgesetzt. Nach säulenchromatographischer Aufarbeitung ergaben sich 946 mg Produkt, 89 % der Theorie.

### Beispiel 15: Molekül 15

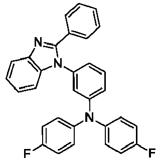

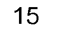

Es wurden 594 mg (1.7 mmol, 1.0 eq) des 1-(3-bromophenyl)-2-phenyl-1H-benzimidazols mit 349 mg (1.7 mmol, 1.0 eq) des entsprechenden Diarylamins versetzt und 4 h bei 80 °C in 50 ml trockenem Toluol umgesetzt. Nach säulenchromatographischer Aufarbeitung ergaben sich 292 mg Produkt, 35 % der Theorie.

### Beispiel 16: Molekül 16

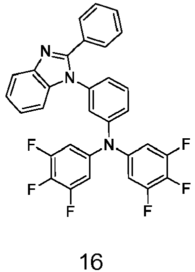

Es wurden 639 mg (1.83 mmol, 1.0 eq) des 1-(3-bromophenyl)-2-phenyl-1H-benzimidazols mit 507 mg (1.83 mmol, 1.0 eq) des entsprechenden Diarylamins versetzt und 4 h bei 80 °C in 50 ml trockenem Toluol umgesetzt. Nach säulenchromatographischer Aufarbeitung ergaben sich 917 mg Produkt, 92 % der Theorie.

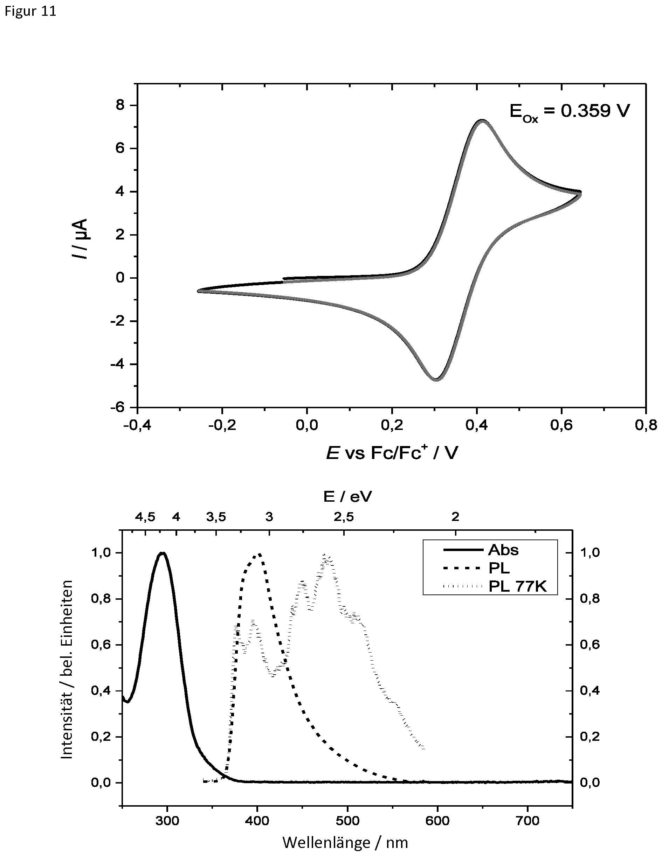

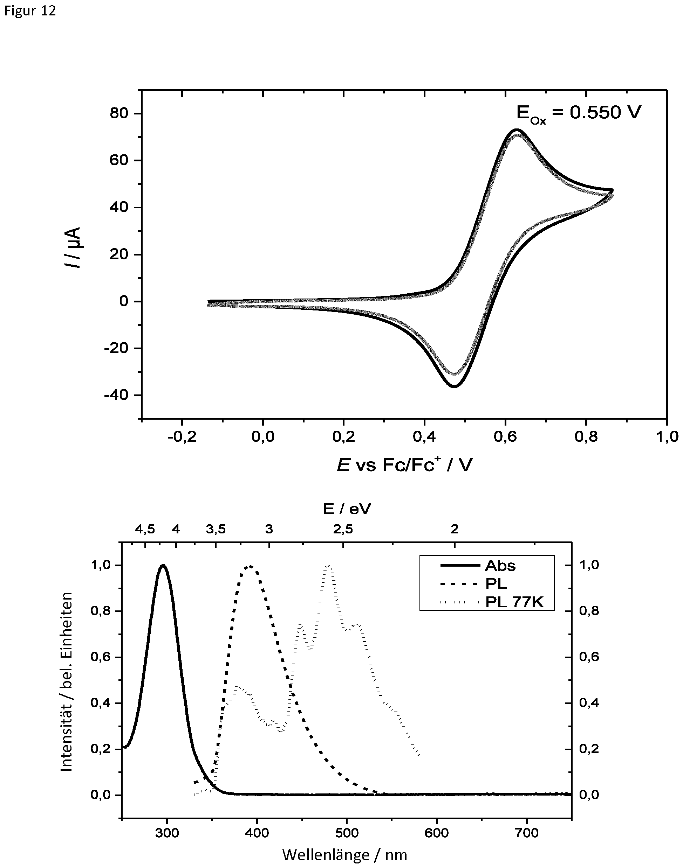

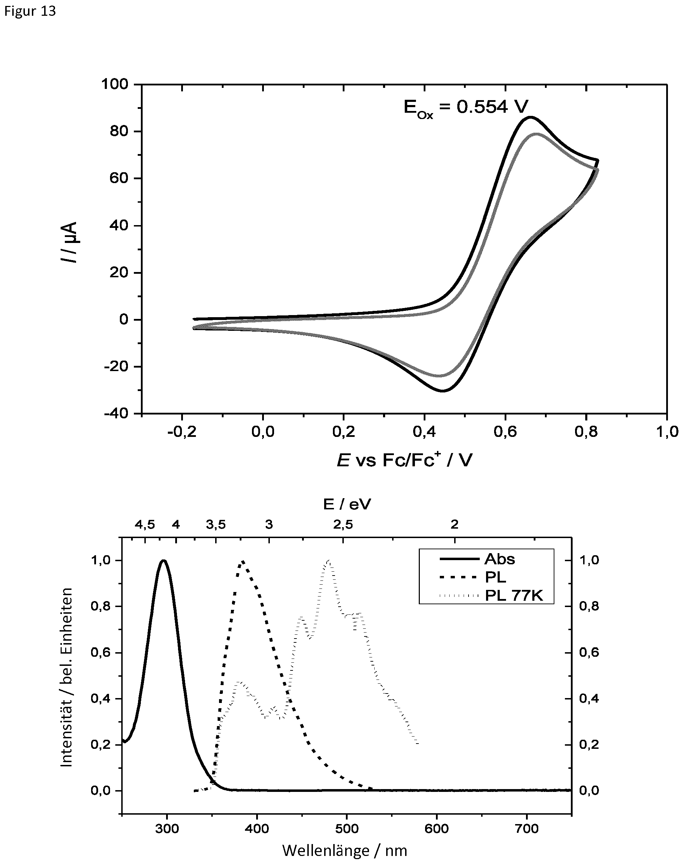

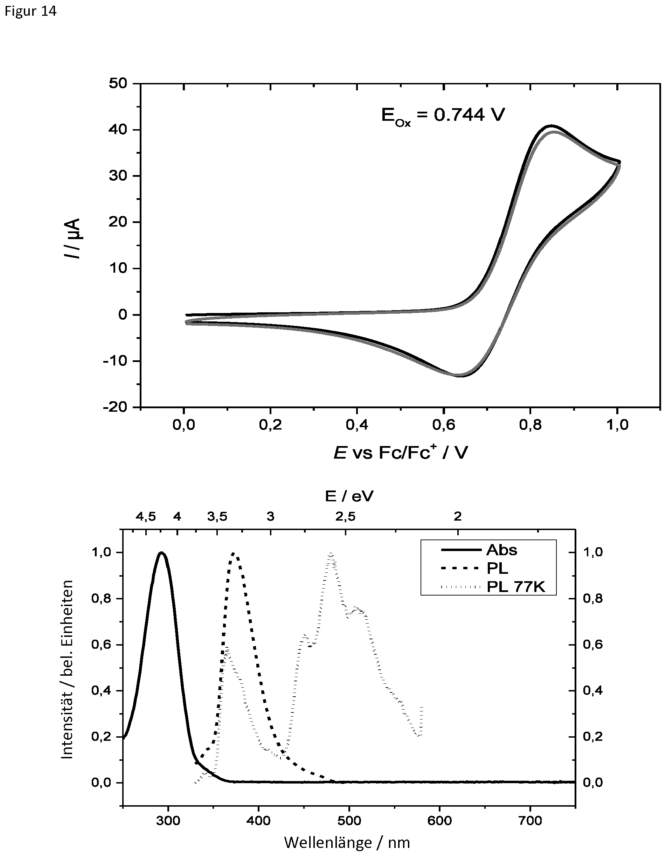

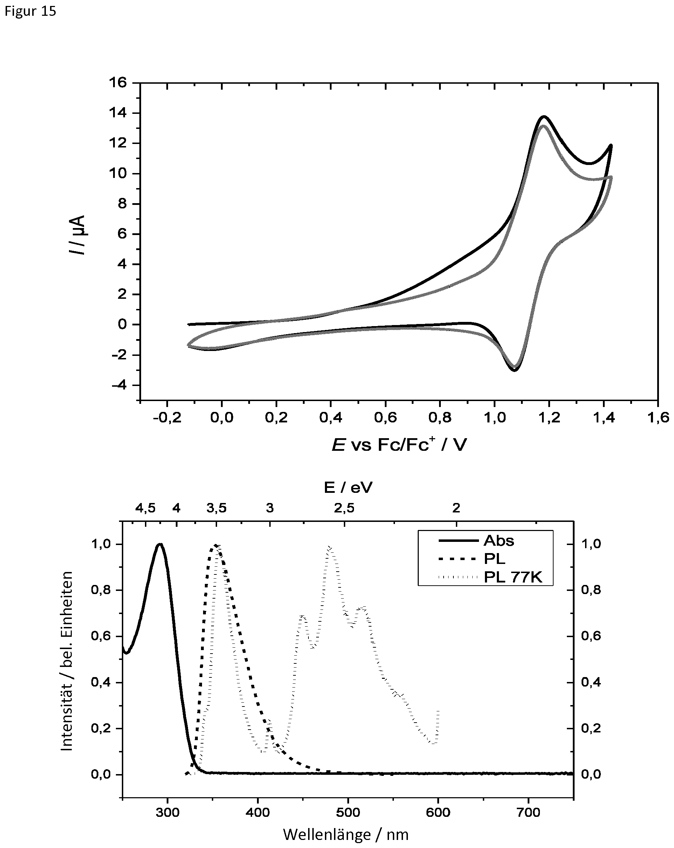

In den Figuren 11 bis 15 sind die elektrochemischen sowie photophysikalischen Messergebnisse für die Moleküle 12 bis 16 dargestellt.

**Tab 4: Es werden das Oxidationspotential gegen Ferrocene gemessen in Volt (E_{Ox} vs Fc / V), die bathochrome Absorptionskante in Nanometer (Abs Icutoff / nm), das Absorptionsmaximum in Nanometer (Abs Imax / nm), die errechnete HOMO Energie in Elektronenvolt (E_{HOMO} / eV), die errechnete LUMO Energie in Elektronenvolt (E_{LUMO} / eV), der S1-Zustand in Elektronenvolt aus der Photolumineszenz bei maximaler Intensität (PLₘₐₓ S1 / eV), der T1-Zustand in Elektronenvolt aus der tieftemperatur Photolumineszenz bei maximaler Intensität (PLₘₐₓ T1 / eV), die Energiedifferenz zwischen S1 und T1 Zustand aus der Photolumineszenz bei maximaler Intensität (DE_{ST, max} / eV), der S1-Zustand in Elektronenvolt aus der hypsochromen Emissionskante der Photolumineszenz (PLₒₙₛₑₜ S1 / eV), der T1-Zustand in Elektronenvolt aus der hypsochromen Emissionskante der tieftemperatur Photolumineszenz (PLₒₙₛₑₜ T1 / eV), die Energiedifferenz zwischen S1 und T1 Zustand aus den hypsochromen Emissionskanten der Photolumineszenz (DE_{ST, max} / eV). Epa ist das anodische Peakpotential der entsprechenden cyclovoltammetrischen Messung, ir steht für einen irreversiblen Elektronentransfer, r für einen reversiblen Elektronentransfer.**

| Material | E_{Ox} vs Fc / V | Abs λ_{cutoff} / nm | Abs λₘₐₓ / nm | E_{HOMO} / eV | E_{LUMO} / eV | PLₘₐₓ S₁ / eV | PLₘₐₓ T₁ / eV | ΔE_{ST} / eV | PLₒₙₛₑₜ S₁/eV | PLₒₙₛₑₜ T₁ / eV | ΔE_{ST, onset} / eV |
|---|---|---|---|---|---|---|---|---|---|---|---|
| 12 | 0.36 V | 332 | 294 | -5.46 | -1.72 | 3.08 | 2.75 | 0.33 | 3,40 | 2,95 | 0,45 |
| 13 | 0.55 V | 332 | 295 | -5.65 | -1.92 | 3.16 | 2.76 | 0.40 | 3,54 | 2,93 | 0,61 |
| 14 | 0.55 V | 332 | 296 | -5.65 | -1.92 | 3.23 | 2.76 | 0.47 | 3,54 | 2,94 | 0,62 |
| 15 | 0.74 V | 327 | 292 | -5.84 | -2.05 | 3.32 | 2.75 | 0.57 | 3,57 | 2,90 | 0,67 |
| 16 | 1.12 V | 325 | 291 | -6.22 | -2.42 | 3.52 | 2.76 | 0.76 | 3,74 | 2,88 | 0,86 |

### Figuren

Figur 1: Cyclovoltammogramm (oben) sowie Absorptions- und Emissionsspektrum (unten) von Molekül 1
Figur 2: Cyclovotlammogramm (oben) sowie Absorbtions- und Emissionssspektrum (unten) für Molekül 2
Figur 3: Cyclovoltammogramm (oben) sowie Absorbtions- und Emissionssspektrum (unten) für Molekül 3
Figur 4: Cyclovoltammogramm (oben) sowie Absorbtions- und Emissionssspektrum (unten) für Molekül 4
Figur 5: Cyclovoltammogramm (oben) sowie Absorbtions- und Emissionssspektrum (unten) für Molekül 5

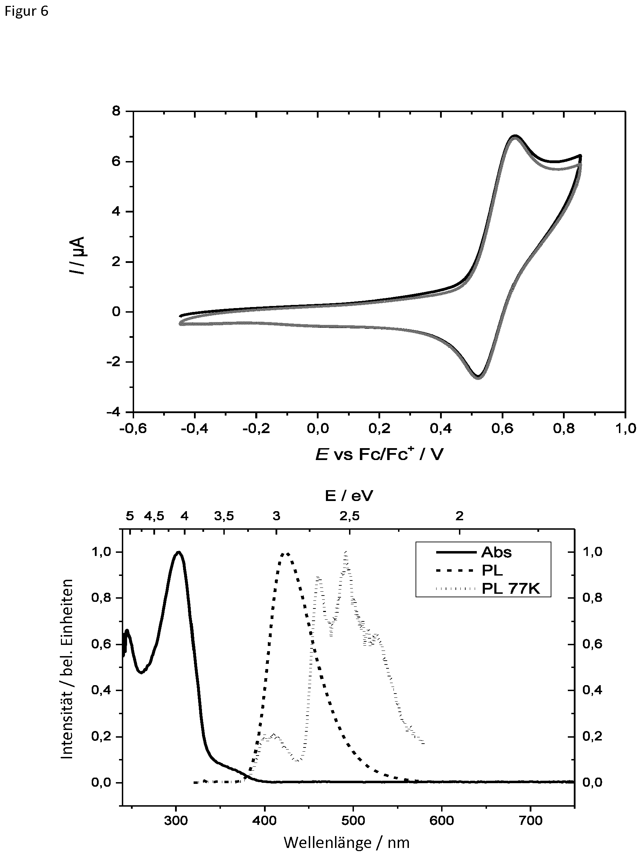

Figur 6: Cyclovoltammogramm (oben) sowie Absorbtions- und Emissionssspektrum (unten) für Molekül 6
Figur 7: Cyclovoltammogramm (oben) sowie Absorptios- und Emissionsspektrum (unten) für Molekül 8
Figur 8: Cyclovoltammogramm (oben) sowie Absorptios- und Emissionsspektrum (unten) für Molekül 9
Figur 9: Cyclovoltammogramm (oben) sowie Absorptios- und Emissionsspektrum (unten) für Molekül 10
Figur 10: Cyclovoltammogramm (oben) sowie Absorptios- und Emissionsspektrum (unten) für Molekül 11
Figur 11: Cyclovoltammogramm (oben) sowie Absorptios- und Emissionsspektrum (unten) für Molekül 12
Figur 12: Cyclovoltammogramm (oben) sowie Absorptios- und Emissionsspektrum (unten) für Molekül 13
Figur 13: Cyclovoltammogramm (oben) sowie Absorptios- und Emissionsspektrum (unten) für Molekül 14
Figur 14: Cyclovoltammogramm (oben) sowie Absorptios- und Emissionsspektrum (unten) für Molekül 15
Figur 15: Cyclovoltammogramm (oben) sowie Absorptios- und Emissionsspektrum (unten) für Molekül 16

## Patentansprüche

1. Organisches Molekül, aufweisend eine Struktur der Formel 1 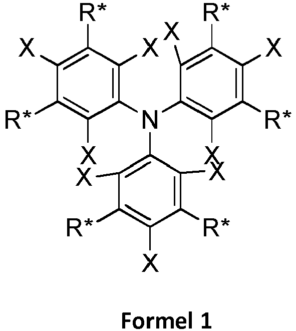
mit R* = Akzeptor-Rest A oder neutraler Rest R, der jeweils unabhängig voneinander über je eine der insgesamt 6 meta-Positionen des Triarylamins angebunden ist, wobei die Zahl an Akzeptor-Resten A mindestens 1 beträgt;
und mit R = bei jedem Auftreten gleich oder verschieden Wasserstoff, Deuterium, Halogen, eine Alkylgruppe (unverzweigt, verzweigt oder zyklisch), eine Alkoxygruppe (unverzweigt, verzweigt oder zyklisch), eine Alkenylgruppe (unverzweigt, verzweigt oder zyklisch), oder eine Aryl- oder Heteroarylgruppe mit 5 bis 40 Ringatomen, die jeweils durch einen oder mehrere Reste R⁴ substituiert sein kann, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 40 aromatischen Ringatomen, die durch einen oder mehrere R⁴ substituiert sein kann, oder eine Aralkyl- oder Heteroaralkylgruppe mit 5 bis 40 aromatischen Ringatomen, die durch einen oder mehrere R⁴ substituiert sein kann, oder eine Kombination dieser Systeme; dabei können zwei oder mehrere Substituenten R auch miteinander, zusammen mit den Atomen, an die sie gebunden sind, ein mono- oder polycylisches aliphatisches oder aromatisches Ringsystem bilden;
R⁴ ist bei jedem Auftreten gleich oder verschieden H, D, OR⁵, SR⁵ oder C(R⁵)₃;
R⁵ ist bei jedem Auftreten gleich oder verschieden Wasserstoff, eine Alkylgruppe oder eine Arylgruppe;
und mit A = Akzeptorgruppe unabhängig voneinander ausgewählt aus der Gruppe bestehend aus 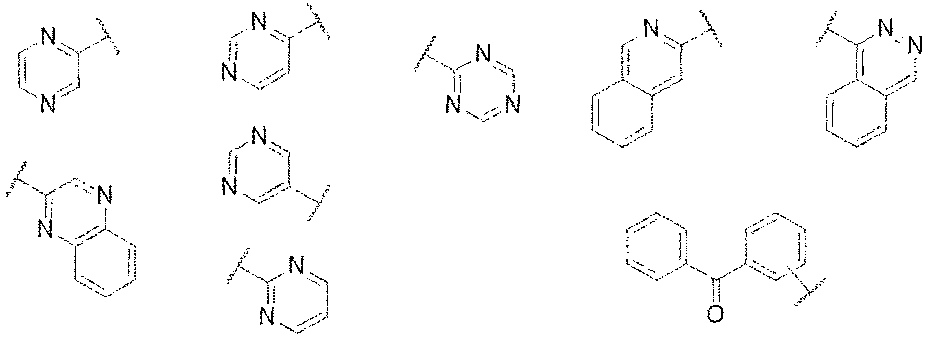 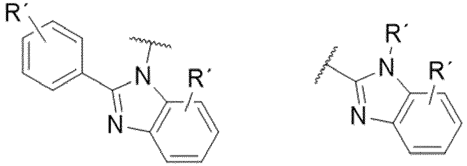 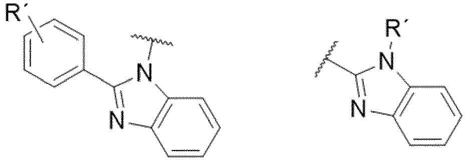 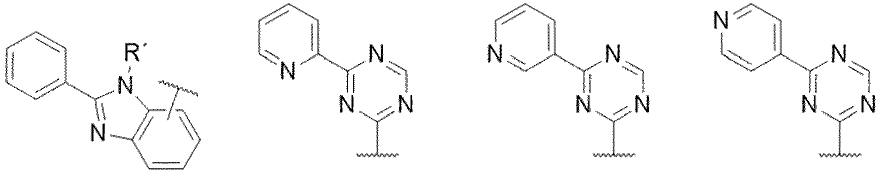 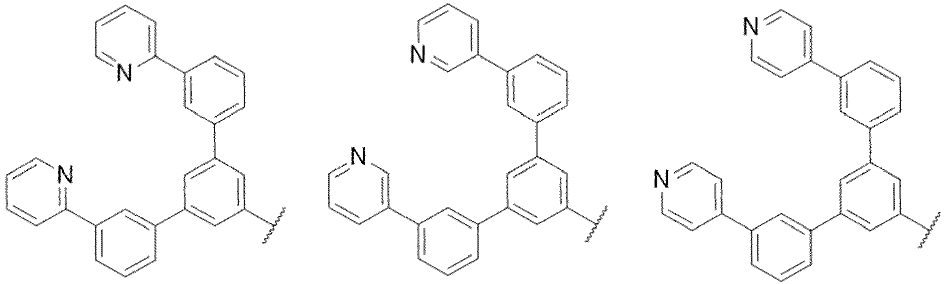 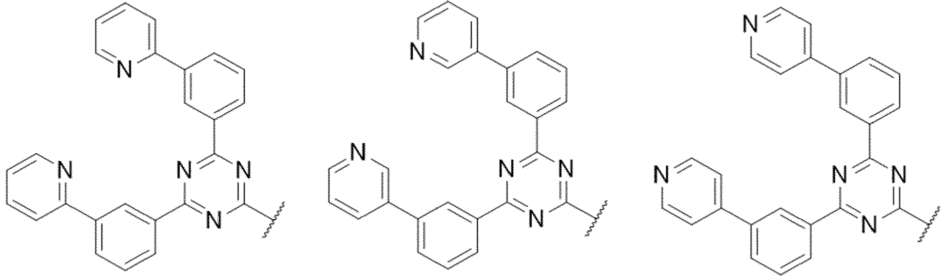 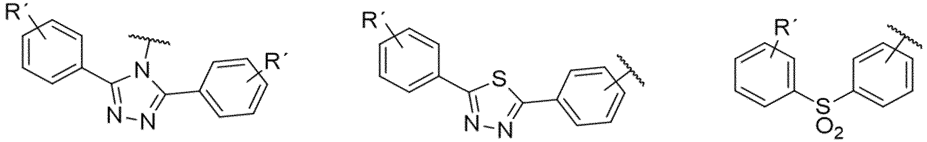 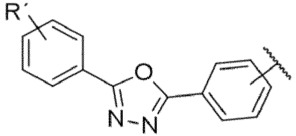 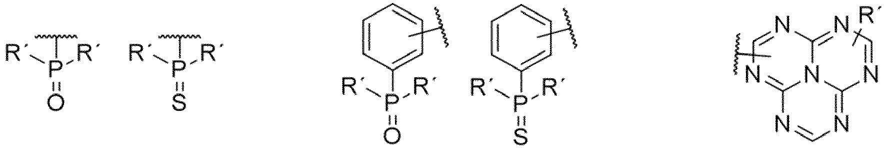
R' ist bei jedem Auftreten gleich oder verschieden
Wasserstoff, Halogen, Deuterium, Gruppen, die über Stickstoff (-NR₂²) oder Sauerstoff- (-OR²) gebunden sind,
Alkyl- (unverzweigt, verzweigt oder zyklisch), Heteroalkyl-, Aryl-, Heteroaryl-, Alkenyl-, Alkinyl-Gruppen oder
substituierte Alkyl- (unverzweigt, verzweigt oder zyklisch), Heteroalkyl-, Aryl-, Heteroaryl- und Alkenyl-Gruppen, die mit Halogen, Deuterium oder Alkylgruppen (unverzweigt, verzweigt oder zyklisch) substituiert sein können;
und mit X = unabhängig voneinander ein neutraler Rest R oder ein Aktivrest Z zur Einstellung der Grenzorbitalenergie der Donoreinheit;
und mit Z = Aktivrest, ausgewählt aus elektronenspendenen Gruppen, die über Stickstoff (-NR₂²) oder Sauerstoff- (-OR²) gebunden sind oder elektronenziehenden Gruppen wie Fluor, Nitril, (C=O)R²;
R² ist bei jedem Auftreten gleich oder verschieden Wasserstoff, Deuterium, Alkyl-(unverzweigt, verzweigt oder zyklisch), Heteroalkyl-, Aryl-, Heteroaryl-, Alkenyl-, Alkinyl-Gruppen oder substituierte Alkyl- (unverzweigt, verzweigt oder zyklisch), Heteroalkyl-, Aryl-, Heteroaryl- und Alkenyl-Gruppen mit Substituenten wie Halogene oder Deuterium, Alkylgruppen (unverzweigt, verzweigt oder zyklisch).

2. Organisches Molekül nach Anspruch 1, wobei die Zahl an Akzeptor-Resten A zwischen 1 und 4 liegen muss und wobei mindestens ein Arylrest des Triaryls keinen Akzeptor trägt

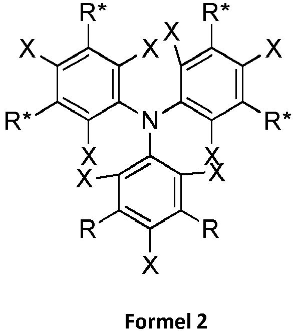

3. Organisches Molekül nach Anspruch 2, wobei Aktivreste Z nur an jenen Arylresten angefügt sind, die nicht gleichzeitig mit Akzeptorresten verknüpft sind (Formel 3a und Formel 3b) 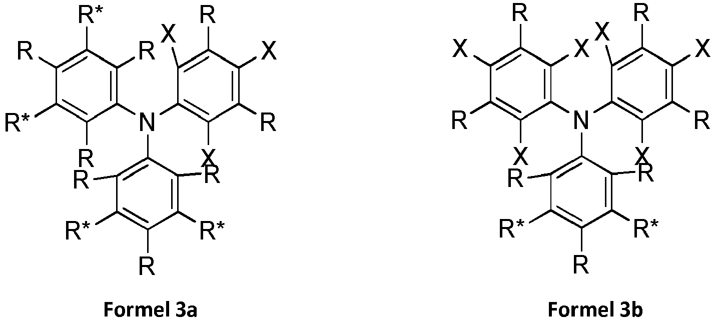 wobei X gleich Z oder R ist.

4. Verwendung eines organischen Moleküls nach Anspruch 1 bis 3 in einem optoelektronischen Bauelement.

5. Verwendung nach Anspruch 4, wobei das organische Molekül in einer emittierenden Schicht, bevorzugt als Matrixmaterial und/oder Sensitizer, insbesondere für lumineszierende Emitter und/oder als Elektroneninjektionsmaterial und/oder Elektronentransportmaterial und/oder als Lochinjektionsmaterial und/oder als Lochtransportmaterial und/oder als Lochblockiermaterial und/oder als Elektronenblockiermaterial verwendet wird.

6. Verwendung nach Anspruch 4 oder 5, wobei das optoelektronische Bauelement ausgewählt ist aus der Gruppe bestehend aus:
- Organischen Licht-emittierenden Bauteilen (OLEDs),
- Licht-emittierenden elektrochemischen Zellen,
- OLED-Sensoren, insbesondere in nicht hermetisch nach außen abgeschirmten Gas- und Dampf-Sensoren,
- organischen Solarzellen,
- Organischen Feldeffekttransistoren,
- Organischen Lasern und
- Down-Konversions-Elementen
- Up-Konversions-Elementen.

7. Optoelektronisches Bauelement, aufweisend ein organisches Molekül nach Anspruch 1 bis 3.

8. Optoelektronisches Bauelement nach Anspruch 7, **dadurch gekennzeichnet, dass** das Organische Molekül nach Anspruch 1 bis 3 in einer emittierenden Schicht eingesetzt ist, bevorzugt als Matrixmaterial insbesondere für lumineszierende Emitter und/oder Elektronentransportmaterial und/oder als Lochinjektionsmaterial und/oder als Lochblockiermaterial.

9. Mischung, enthaltend mindestens ein organisches Molekül nach Anspruch 1 bis 3 und mindestens einen lumineszierenden Emitter.

10. Formulierung, enthaltend mindestens ein organisches Molekül nach Anspruch 1 bis 3 oder eine Mischung nach Anspruch 9 und mindestens ein Lösemittel.

11. Verfahren zur Herstellung eines optoelektronischen Bauelements, wobei ein organisches Molekül nach Anspruch 1 bis 3 verwendet wird.

12. Verfahren nach Anspruch 11, **gekennzeichnet durch** das Aufbringen eines organischen Moleküls nach Anspruch 1 bis 3 auf einen Träger.

13. Verfahren nach Anspruch 12, **dadurch gekennzeichnet, dass** das Aufbringen nass-chemisch erfolgt.

14. Verfahren nach Anspruch 12, **dadurch gekennzeichnet, dass** das Aufbringen durch Verdampfung erfolgt.

## Claims

1. An organic molecule, comprising a structure of Formula 1 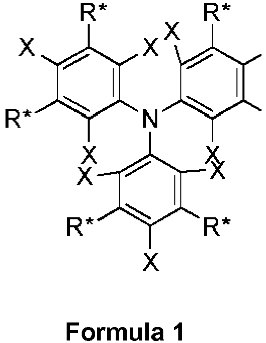
where R* = acceptor group A or neutral group R, which in each case independently of each other is linked via one each of the total of 6 meta positions of the triarylamine, wherein the number of acceptor groups A is at least 1;
and where R = is for each occurrence the same or different hydrogen, deuterium, halogen, an alkyl group (unbranched, branched or cyclic), an alkoxy group (unbranched, branched or cyclic), an alkenyl group (unbranched, branched or cyclic), or an aryl or heteroaryl group with 5 to 40 ring atoms, which may be substituted in each case by one or more groups R⁴, or an aromatic or heteroaromatic ring system with 5 to 60 aromatic ring atoms which may be substituted in each case by one or more groups R⁴, or an aryloxy or heteroaryloxy group with 5 to 40 aromatic ring atoms which may be substituted by one or more R⁴, or an aralkyl group or heteroaralkyl group with 5 to 40 aromatic ring atoms which may be substituted by one or more R⁴, or a combination of these systems; wherein two or more substituents R may also jointly, together with the atoms to which they are bonded, form a monocyclic or polycylic aliphatic or aromatic ring system;
R⁴ is for each occurrence the same or different H, D, OR⁵, SR⁵ or C(R⁵)₃;
R⁵ is for each occurrence the same or different hydrogen, an alkyl group or an aryl group;
and where A = acceptor group is independently of each other selected from the group consisting of 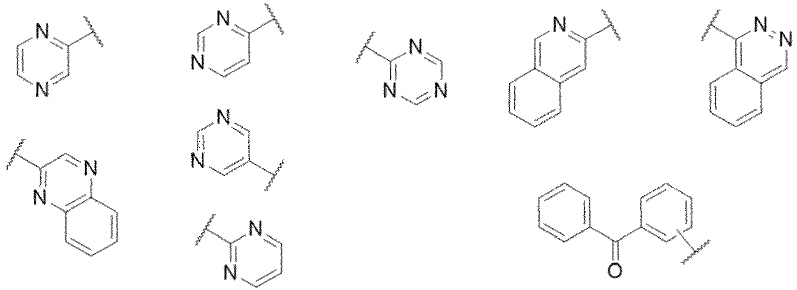 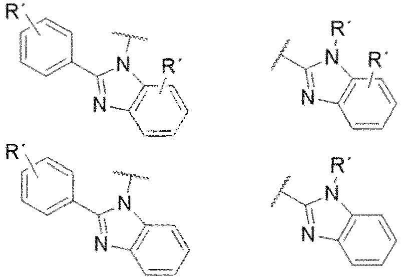 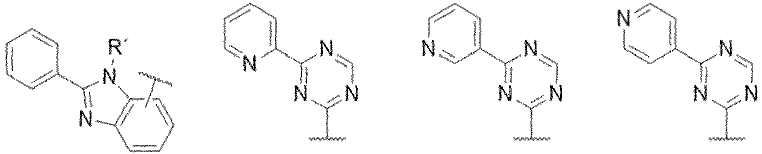 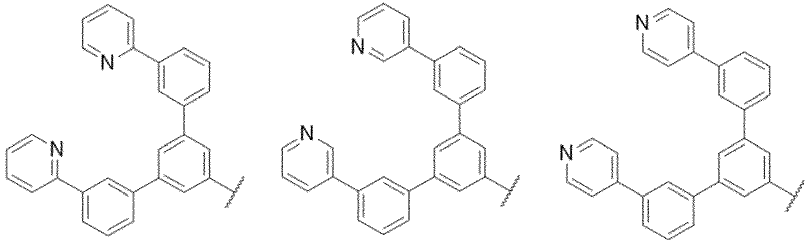 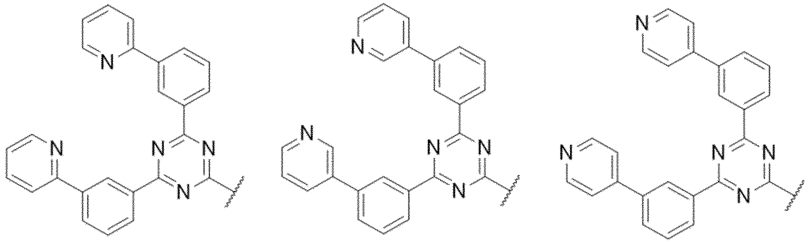 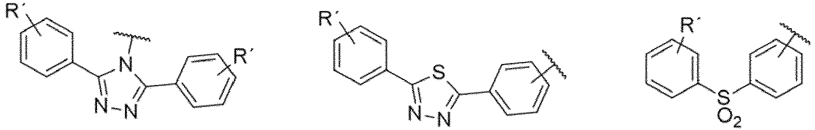 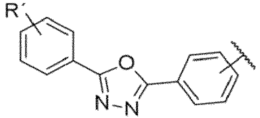 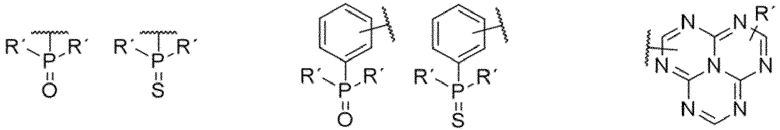
R' is for each occurrence the same or different
hydrogen, halogen, deuterium, groups which are bonded via nitrogen (-NR₂²) or oxygen (-OR²),
alkyl (unbranched, branched or cyclic), heteroalkyl, aryl, heteroaryl, alkenyl, alkynyl groups, or
substituted alkyl (unbranched, branched or cyclic), heteroalkyl, aryl, heteroaryl and alkenyl groups which may be substituted with halogen, deuterium or alkyl groups (unbranched, branched or cyclic);
and where X = independently of each other a neutral group R or an active group Z for setting the frontier orbital energy of the donor unit;
and where Z = active group, selected from electron-donating groups which are bonded via nitrogen (-NR₂²) or oxygen (-OR²) or electron-withdrawing groups such as fluorine, nitrile, (C=O)R²;
R² is for each occurrence the same or different hydrogen, deuterium, alkyl groups (unbranched, branched or cyclic), heteroalkyl groups, aryl groups, heteroaryl groups, alkenyl groups, alkynyl groups or substituted alkyl groups (unbranched, branched or cyclic), heteroalkyl groups, aryl groups, heteroaryl groups and alkenyl groups with substituents such as halogens or deuterium, alkyl groups (unbranched, branched or cyclic).

2. The organic molecule according to Claim 1, wherein the number of acceptor groups A is between 1 and 4 and wherein at least one aryl group of the triaryl does not bear an acceptor

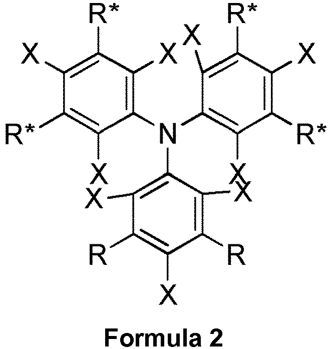

3. The organic molecule according to Claim 2, wherein active groups Z are only attached to those aryl groups which are not at the same time linked to acceptor groups (Formula 3a and Formula 3b) 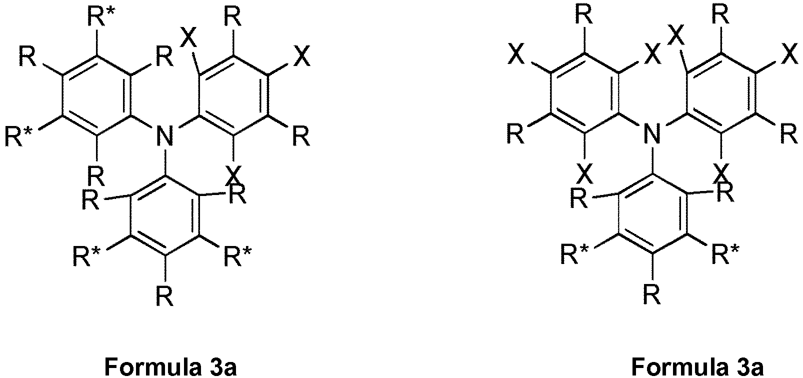 wherein X is equal to Z or R.

4. Use of an organic molecule according to Claim 1 to 3 in an optoelectronic device.

5. Use according to Claim 4, wherein the organic molecule is used in an emitting layer, preferably as a matrix material and/or sensitizer, in particular for luminescent emitters and/or as electron injection material and/or electron transport material and/or as hole injection material and/or as hole transport material and/or as hole blocking material and/or as electron blocking material.

6. Use according to Claim 4 or 5, wherein the optoelectronic device is selected from the group consisting of:
- organic light-emitting devices (OLEDs),
- light-emitting electrochemical cells,
- OLED sensors, in particular in gas and vapour sensors which are not hermetically shielded from the outside,
- organic solar cells,
- organic field-effect transistors,
- organic lasers and
- down-conversion elements
- up-conversion elements.

7. An optoelectronic device, comprising an organic molecule according to Claims 1 to 3.

8. The optoelectronic device according to Claim 7, **characterised in that** the organic molecule according to Claims 1 to 3 is used in an emitting layer, preferably as a matrix material in particular for luminescent emitters and/or electron transport material and/or as hole injection material and/or as hole blocking material.

9. A mixture, comprising at least an organic molecule according to Claims 1 to 3 and at least a luminescent emitter.

10. A formulation, comprising at least an organic molecule according to Claims 1 to 3 or a mixture according to Claim 9 and at least a solvent.

11. A method for producing an optoelectronic device, wherein an organic molecule according to Claims 1 to 3 is used.

12. The method according to Claim 11, **characterised by** the application of an organic molecule according to Claims 1 to 3 to a support.

13. The method according to Claim 12, **characterised in that** the application takes place by a wet-chemical process.

14. The method according to Claim 12, **characterised in that** the application takes place by evaporation.

## Revendications

1. Molécule organique, comprenant une structure de la formule 1 : 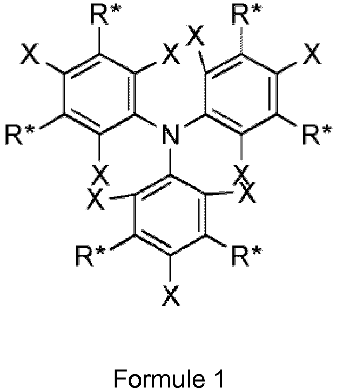
avec les R* = radical accepteur A ou radical neutre R, qui sont à chaque fois indépendamment les uns des autres reliés par une de toutes les 6 positions méta de la triarylamine, le nombre de radicaux accepteurs A étant d'au moins 1 ;
et avec les R = à chaque occurrence de manière identique ou différente, hydrogène, deutérium, halogène, un groupe alkyle (non ramifié, ramifié ou cyclique), un groupe alcoxy (non ramifié, ramifié ou cyclique), un groupe alcényle (non ramifié, ramifié ou cyclique) ou un groupe aryle ou hétéroaryle de 5 à 40 atomes de cycle, qui peut à chaque fois être substitué par un ou plusieurs radicaux R⁴, ou un système cyclique aromatique ou hétéroaromatique de 5 à 60 atomes de cycle aromatique, qui peut à chaque fois être substitué par un ou plusieurs radicaux R⁴, ou un groupe aryloxy ou hétéroaryloxy de 5 à 40 atomes de cycle aromatique, qui peut être substitué par un ou plusieurs R⁴, ou un groupe aralkyle ou hétéroaralkyle de 5 à 40 atomes de cycle aromatique, qui peut être substitué par un ou plusieurs R⁴, ou une combinaison de ces systèmes, deux substituants R ou plus pouvant également former les uns avec les autres, conjointement avec les atomes auxquels ils sont reliés, un système cyclique mono- ou polycyclique, aliphatique ou aromatique ;
les R⁴ représentent, à chaque occurrence de manière identique ou différente, H, D, OR⁵, SR⁵ ou C(R⁵)₃;
les R⁵ représentent, à chaque occurrence de manière identique ou différente, l'hydrogène, un groupe alkyle ou un groupe aryle ;
et avec les A = groupes accepteurs choisis indépendamment les uns des autres dans le groupe constitué par : 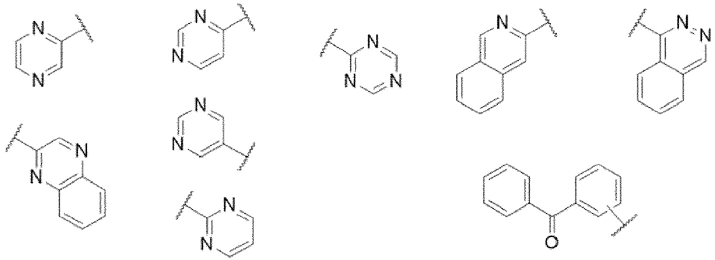 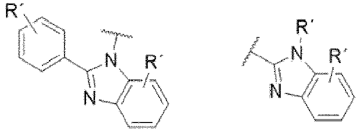 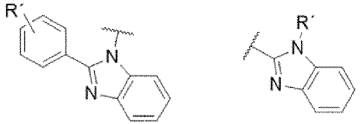 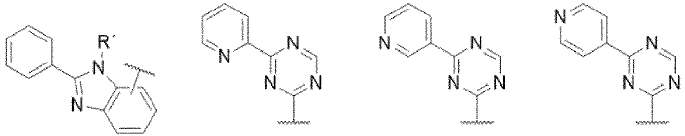 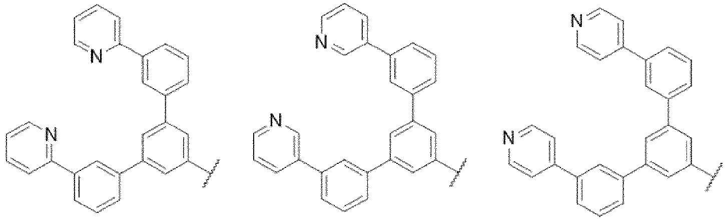 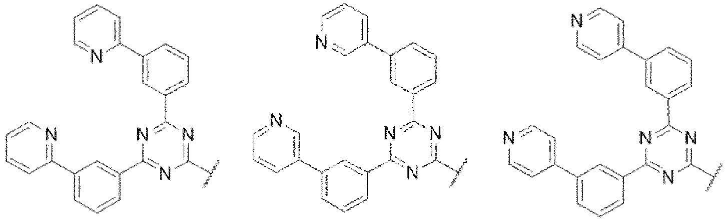 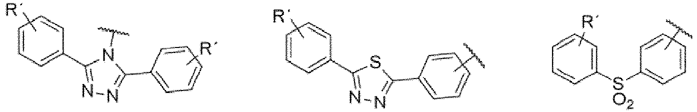 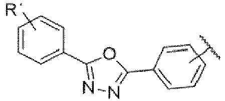 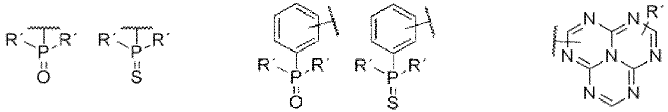
les R' représentant, à chaque occurrence de manière identique ou différente :
hydrogène, halogène, deutérium, des groupes qui sont reliés par un azote (-NR₂²) ou un oxygène (-OR²),
des groupes alkyle (non ramifiés, ramifiés ou cycliques), hétéroalkyle, aryle, hétéroaryle, alcényle, alcynyle, ou
des groupes alkyle (non ramifiés, ramifiés ou cycliques), hétéroalkyle, aryle, hétéroaryle et alcényle substitués, qui peuvent être substitués avec halogène, deutérium ou des groupes alkyle (non ramifiés, ramifiés ou cycliques) ;
et avec les X = indépendamment les uns des autres, un radical neutre R ou un radical actif Z pour l'ajustement de l'énergie orbitale limite de l'unité donneuse ;
et avec Z = radical actif, choisi parmi les groupes donneurs d'électrons qui sont reliés par un azote (-NR₂²) ou un oxygène (-OR²) ou les groupes attracteurs d'électrons tels que fluor, nitrile, (C=O)R² ;
les R² représentant, à chaque occurrence indépendamment les uns des autres, hydrogène, deutérium, des groupes alkyle (non ramifiés, ramifiés ou cycliques), hétéroalkyle, aryle, hétéroaryle, alcényle, alcynyle, ou des groupes alkyle (non ramifiés, ramifiés ou cycliques), hétéroalkyle, aryle, hétéroaryle et alcényle substitués avec des substituants tels que des halogènes ou le deutérium, des groupes alkyle (non ramifiés, ramifiés ou cycliques).

2. Molécule organique selon la revendication 1, dans laquelle le nombre de radicaux accepteurs A doit être compris entre 1 et 4, et dans laquelle au moins un radical aryle du triaryle ne porte pas d'accepteur :

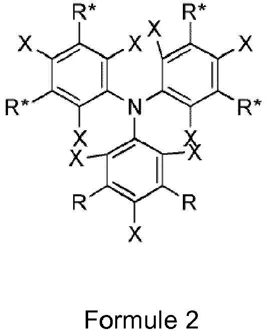

3. Molécule organique selon la revendication 2, dans laquelle des radicaux actifs Z ne sont reliés qu'aux radicaux aryle qui ne sont pas simultanément reliés avec des radicaux accepteurs (formule 3a et formule 3b) : 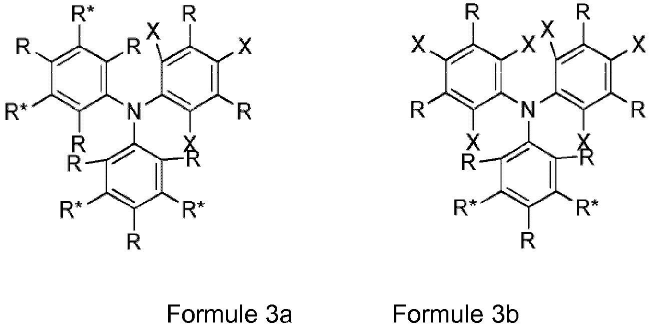 dans lesquelles X représente Z ou R.

4. Utilisation d'une molécule organique selon les revendications 1 à 3 dans un composant optoélectronique.

5. Utilisation selon la revendication 4, dans laquelle la molécule organique est utilisée dans une couche d'émission, de préférence en tant que matériau de matrice et/ou sensibilisateur, notamment pour un émetteur luminescent et/ou en tant que matériau d'injection d'électrons et/ou matériau de transport d'électrons et/ou en tant que matériau d'injection de trous et/ou en tant que matériau de transport de trous et/ou en tant que matériau de blocage de trous et/ou en tant que matériau de blocage d'électrons.

6. Utilisation selon la revendication 4 ou 5, dans laquelle le composant optoélectronique est choisi dans le groupe constitué par :
- les composants électroluminescents organiques (OLED),
- les cellules électrochimiques électroluminescentes,
- les capteurs OLED, notamment dans des capteurs de gaz et de vapeur non blindés hermétiquement vers l'extérieur,
- les cellules solaires organiques,
- les transistors à effet de champ organiques,
- les lasers organiques et
- les éléments de conversion à la baisse,
- les éléments de conversion à la hausse.

7. Composant optoélectronique, comprenant une molécule organique selon les revendications 1 à 3.

8. Composant optoélectronique selon la revendication 7, **caractérisé en ce que** la molécule organique selon les revendications 1 à 3 est utilisée dans une couche d'émission, de préférence en tant que matériau de matrice, notamment pour un émetteur luminescent et/ou un matériau de transport d'électrons et/ou en tant que matériau d'injection de trous et/ou en tant que matériau de blocage de trous.

9. Mélange, contenant au moins une molécule organique selon les revendications 1 à 3 et au moins un émetteur luminescent.

10. Formulation, contenant au moins une molécule organique selon les revendications 1 à 3 ou un mélange selon la revendication 9 et au moins un solvant.

11. Procédé de fabrication d'un composant optoélectronique, dans lequel une molécule organique selon les revendications 1 à 3 est utilisée.

12. Procédé selon la revendication 11, **caractérisé par** l'application d'une molécule organique selon les revendications 1 à 3 sur un support.

13. Procédé selon la revendication 12, **caractérisé en ce que** l'application a lieu par voie chimique humide.

14. Procédé selon la revendication 12, **caractérisé en ce que** l'application a lieu par évaporation.
